(19) **Europäisches Patentamt / European Patent Office / Office européen des brevets**

(11) **EP 4 436 948 B1**

(12) **EUROPÄISCHE PATENTSCHRIFT**

(45) Veröffentlichungstag und Bekanntmachung des Hinweises auf die Patenterteilung:
**06.08.2025 Patentblatt 2025/32**

(21) Anmeldenummer: **22817916.4**

(22) Anmeldetag: **14.11.2022**

(51) Internationale Patentklassifikation (IPC):
**C07C 67/08** (2006.01)    **C07C 67/54** (2006.01)
**C07C 69/54** (2006.01)

(52) Gemeinsame Patentklassifikation (CPC):
(C-Sets verfügbar)
**C07C 67/08; C07C 67/54**    (Forts.)

(86) Internationale Anmeldenummer:
**PCT/EP2022/081719**

(87) Internationale Veröffentlichungsnummer:
**WO 2023/094190 (01.06.2023 Gazette 2023/22)**

(54) **VERFAHREN ZUR HERSTELLUNG VON 2-OCTYL(METH)ACRYLAT**

METHOD FOR THE PREPARATION OF 2-OCTYL(METH)ACRYLATE

PROCÉDÉ DE PRODUCTION DE 2-OCTYL(MÉTH)ACRYLATE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **25.11.2021 EP 21210376**

(43) Veröffentlichungstag der Anmeldung:
**02.10.2024 Patentblatt 2024/40**

(73) Patentinhaber: **BASF SE**
**67056 Ludwigshafen am Rhein (DE)**

(72) Erfinder:
• **MISSKE, Andrea**
  **67056 Ludwigshafen (DE)**
• **LANG, Ortmund**
  **67056 Ludwigshafen (DE)**
• **DECKERT, Petra**
  **67056 Ludwigshafen (DE)**

• **DE RUITER, Cornelis Hendricus**
  **67056 Ludwigshafen (DE)**
• **AKIN, Aykan**
  **Shanghai 200137 (CN)**
• **KORPJUHN, Frank**
  **67056 Ludwigshafen (DE)**
• **FLECKENSTEIN, Christoph**
  **67056 Ludwigshafen (DE)**
• **FLEISCHHAKER, Friederike**
  **67056 Ludwigshafen (DE)**
• **EICHHORN, Sabine**
  **67056 Ludwigshafen (DE)**

(74) Vertreter: **BASF IP Association**
**BASF SE**
**GBI - Z078**
**67056 Ludwigshafen (DE)**

(56) Entgegenhaltungen:
**WO-A1-2008/046000    WO-A1-2013/064775**
**CN-A- 1 298 863    GB-A- 962 928**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C07C 67/08, C07C 69/54;**
**C07C 67/54, C07C 69/54**

**Beschreibung**

Die vorliegende Erfindung betrifft ein Verfahren zur Herstellung von 2-Octyl(meth)acrylat durch Umsetzung von 2-Octanol mit (Meth)acrylsäure in Gegenwart eines sauren Veresterungskatalysators, eines Polymerisationsinhibitors und des Schleppmittels Cyclohexan.

Der Begriff (Meth)acrylsäure steht in dieser Schrift verkürzend für Methacrylsäure und/oder Acrylsäure. Analog steht 2-Octyl(meth)acrylat für 2-Octylacrylat und/oder 2-Octylmethacrylat.

2-Octyl(meth)acrylat ist ein Monomer, das in radikalischen, kat- und anionischen sowie komplexkatalysierten Polymerisationen als Homo- und Co-Monomer genutzt werden kann. Es eignet sich sowohl für den Einsatz in Emulsationspolymerisationen als auch lösemittelbasierten Polymerisationen oder Massepolymerisationen.

Wichtige Anwendungen sind beispielsweise dekorative Beschichtungen oder technische Beschichtungen für den Industriebereich sowie Klebstoffe, Dichtstoffe, Papierchemikalien, wie z.B. Binder und Leimungsmittel, Leder- und Textilchemikalien, wie z.B. Binder und Hydrophobisierungsmittel, Rheologieaddditive, Impact Modifier für Kunststoffe, Pour Point Depressants für Öl und Schmierstoffe, Druckfarben oder Reaktivverdünner für UV-härtende Harze und Systeme.

Bekannte Verfahren zur großtechnischen Herstellung von 2-Octyl(meth)acrylat basieren überwiegend auf der Umesterungsreaktion zwischen einem (Meth)acrylat eines niederen Alkohols und 2-Octanol in Gegenwart eines Alkyltitanats als Umesterungskatalysator und zumindest eines Polymerisationsinhibitors. Solch ein Verfahren wird in WO 2013/110877 A1 (Arkema France) offenbart. Nachteilig an den Umesterungsverfahren ist der Anfall eines Azeotrops aus dem Alkohol des niedersiedenden (Meth)acrylats und dem niedersiedenden (Meth)acrylat, beispielsweise aber nicht einschränkend Methanol/Methylmethacrylat oder Ethanol/Ethylacrylat, das aufwendig aufgearbeitet oder entsorgt werden muss.

Ein Verfahren zur direkten Veresterung von 2-Octylacrylat offenbart WO 2013/064775 A1 (Arkema France). Durch die direkte Veresterung von Acrylsäure mit 2-Octanol in Gegenwart eines Katalysators sowie eines Polymerisationsinhibitors wird das Wertprodukt 2-Octylacrylat gewonnen, wobei das durch die Veresterung gebildete Veresterungswasser mit dem 2-Octanol ein heterogenes Azeotrop bildet und durch Destillation in einer auf den Reaktor aufgesetzten Destillationskolonne abgetrennt wird. Der Reaktor ist hierbei mit einem Rührer sowie mit einem externen Wärmeübertrager ausgestattet. In einem nachgeschalteten Reinigungsschritt wird zumindest teilweise der Katalysator dem Reaktor zurückgeführt und das Wertprodukt 2-Octylacrylat separiert.

Ein weiteres Verfahren zur direkten Veresterung von 2-Octylacrylat offenbart WO 2008/046000 A1 (3M Innovative Properties Company, USA). Durch die direkte Veresterung von Acrylsäure mit 2-Octanol in Gegenwart des Katalysators p-Toluolsulfonsäure, des Polymerisationsinhibitors Phenothiazin sowie des Schleppmittels Toluol wird das Wertprodukt 2-Octylacrylat gewonnen, wobei das durch die Veresterung gebildete Veresterungswasser mit dem Schleppmittel Toluol ein heterogenes Azeotrop bildet und dieses heterogenes Azeotrop durch Destillation in einer auf den Reaktor aufgesetzten "Dean Stark" Destillationskolonne getrennt wird.

Diese beiden vorstehend beschriebenen Verfahren zur direkten Veresterung von 2-Octylacrylat haben insbesondere den Nachteil, dass ein hoher Energieaufwand benötigt wird, weil das bei der Veresterung entstehende Veresterungswasser durch Verdampfung aus dem Reaktor herausgeführt werden muss, um das verdampfte Veresterungswasser in einer dem Reaktor aufgesetzten Destillationskolonne abtrennen zu können. Somit muss die hohe Siedetemperatur von Wasser oder die hohe Siedetemperatur des heterogenen Azeotrops "Veresterungswasser mit Toluol und/oder Veresterungswasser mit 2-Octanol im Überschuss" im Sumpf des Reaktors (1) nicht nur erreicht, sondern sogar übertroffen werden, damit am Kopf der Destillationskolonne die benötigte Siedetemperatur erzielt werden kann.

Es stellte sich daher die Aufgabe ein Verfahren zur Herstellung von 2-Octyl(meth)acrylat bereitzustellen, welches mit einem niedrigeren Energieaufwand, bei einer niedrigeren Sumpftemperatur im Reaktor und bei moderaten Drücken, wie z.B. bei Normaldruck, betrieben werden kann, ohne dass hierbei aufwendige Apparate verwendet werden müssen. Zudem sollte das Verfahren bei gleichem Energieeinsatz eine bessere Raum-Zeit Ausbeute erreichen als die bekannten Verfahren aus dem vorstehend zitierten Stand der Technik.

Diese Aufgabe wird gemäß der vorliegenden Erfindung durch ein Verfahren zur Herstellung von 2-Octyl(meth) acrylat nach Anspruch 1 gelöst. Vorteilhafte Ausführungsformen des Verfahrens sind in den Ansprüchen 2 bis 15 wiedergegeben.

Das erfindungsgemäße Verfahren zur Herstellung von 2-Octyl(meth)acrylat durch Umsetzung von 2-Octanol mit (Meth)acrylsäure in Gegenwart eines sauren Veresterungskatalysators, eines Polymerisationsinhibitors und des Schleppmittels Cyclohexan umfasst die Schritte:

- Bereitstellen einer Reaktoreinheit (24), wobei sich ein Reaktor (1) mit einem Reaktorheizelement (30) innerhalb der Reaktoreinheit (24) befindet,

- Zuführen von 2-Octanol, (Meth)acrylsäure, saurem Veresterungskatalysator, Cyclohexan und Polymerisationsinhibitor in den einen Reaktor (1),

- Durchführen einer Veresterung in dem einen Reaktor (1) unter Bildung eines flüssigen Reaktionsgemischs, wobei die Veresterung in dem einen Reaktor (1) bei einer Sumpftemperatur im Bereich von 90 bis 130 °C und bei einem Absolutdruck im Bereich von 0,5 bis 2,0 bar stattfindet, und ein resultierender Reaktionsaustrag aus dem einen Reaktor (1) erhalten wird, wobei der resultierende Reaktionsaustrag zumindest 2-Octyl(meth)acrylat, 2-Octanol, (Meth)acrylsäure, sauren Veresterungskatalysator, Cyclohexan, Veresterungswasser und Polymerisationsinhibitor enthält, und das bei der Veresterung entstehende Veresterungswasser zusammen mit dem Schleppmittel Cyclohexan ein heterogenes Azeotrop bildet,

- Verdampfen des heterogenen Azeotrops aus dem flüssigen Reaktionsgemisch des einen Reaktors (1), wobei das Verdampfen durch das eine Reaktorheizelement (30) erreicht wird, und

- Abtrennen des gasförmigen heterogenen Azeotrops aus dem einen Reaktor (1),
  wobei das gasförmige heterogene Azeotrop in einem Kondensator (5) kondensiert und anschließend einem Phasenscheider (6) zugeführt wird und das Veresterungswasser als untere Phase und das Cyclohexan als obere Phase in diesem Phasenscheider (6) abgetrennt wird.

In dieser Schrift dienen die Bezugszeichen in Klammern einem besseren Verständnis beim Lesen. Die Bezugszeichen in Klammern wirken nicht einschränkend, sondern stellen nur jeweils ein mögliches Beispiel von mehreren Umsetzungsmöglichkeiten dar.

Im Folgenden werden die einzelnen Verfahrensstufen von I bis VII beschrieben, wobei die Verfahrensstufen II bis VII optional zu betrachten sind.

Verfahrensstufe I: Veresterung

Das erfindungsgemäße Verfahren basiert auf den Edukten 2-Octanol und (Meth)acrylsäure. Als (Meth)acrylsäure wird in dieser Schrift eine (Meth)acrylsäure-Qualität bezeichnet, die bevorzugt mindestens 98 Gew.-%, weiter bevorzugt mit mindestens 99,5 Gew.-%, (Meth)acrylsäure , daneben bevorzugt maximal 0,2 Gew.-% Wasser sowie jeweils bevorzugt maximal 0,03 Gew.-% Essigsäure, Propionsäure und Isobuttersäure aufweist. Vorzugsweise wird eine 2-Octanol-Qualität mit mindestens 99 % Gew.-% 2-Octanol, maximal 0,1 % 2-Octanon, maximal 0,3 % 1-Heptanol, maximal 0,3 % Octenol (cis/trans), maximal 0,1 % anderen Alkoholen sowie maximal 0,5 % Wasser eingesetzt. Die Farbzahl beträgt vorzugsweise maximal APHA 15, die Säurezahl maximal 0,2 mgKOH/g.

Geeignete Polymerisationsinhibitoren, die als Stabilisatoren wirken, können beispielsweise N-Oxide (Nitroxyl- oder N-Oxyl-Radikale, also Verbindungen, die wenigstens eine NO-Gruppe aufweisen), wie z. B. 4-Hydroxy-2,2,6,6-tetramethylpiperidin-N-oxyl (HO-TEMPO), 4-Oxo-2,2,6,6-tetramethyl-piperidin-N-oxyl, 4-Acetoxy-2,2,6,6-tetramethyl-piperidin-N-oxyl, 2,2,6,6-Tetra- methylpiperidin-N-oxyl, Bis(1-oxyl-2,2,6,6-tetramethyl-piperidine-4-yl)sebacat, 4,4',4"-Tris(2,2,6,6-tetramethyl-piperidin-N-oxyl)-phosphit oder 3-Oxo-2,2,5,5-tetramethyl-pyrrolidin-N-oxyl; ein- oder mehrwertige Phenole, die ggf. eine oder mehrere Alkylgruppen aufweisen, wie z. B. Alkylphenole, beispielsweise o-, m- oder p-Kresol (Methylphenol), 2-tert.-Butylphenol, 4-tert.-Butylphenol, 2,4-Di-tert.-butylphenol, 2-Methyl-4-tert.-butyl-phenol, 2-tert.-Butyl-4-methylphenol, 2,6-tert.-Butyl-4-methylphenol, 4-tert.-Butyl-2,6-dimethylphenol oder 6- tert.-Butyl-2,4-dimethylphenol; Chinone, wie z. B. Hydrochinon, Hydrochinonmonomethylether, 2-Methylhydro- chinon oder 2,5-Di-tert.-Butylhydrochinon; Hydroxyphenole, wie beispielsweise Brenzcatechin (1,2-Dihydroxy-benzol) oder Benzochinon; Aminophenole, wie z. B. p-Aminophenol; Nitrosophenole, wie z. B. p-Nitrosophenol; Alkoxyphenole, wie beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Ditert.-Butyl-4-methoxyphenol; Tocopherole, wie z. B. $\alpha$-Tocopherol sowie 2,3-Dihydro-2,2-dimethyl-7-hydroxybenzofuran (2,2-Dimethyl-7-hydroxycumaran), aromatische Amine, wie z. B. N,N-Diphenylamin oder N-Nitroso-diphenyl-amin; Phenylendiamine, wie z. B. N,N'-Dialkyl-p-phenylendiamin, wobei die Alkylreste gleich oder verschieden sein können und jeweils unabhängig voneinander aus 1 bis 4 Kohlenstoffatomen bestehen und geradkettig oder verzweigt sein können, wie z. B. N,N'-Dimethyl-p-phenylendiamin oder N,N'-Diethyl-p-phenylendiamin, Hydroxylamine, wie z. B. N,N-Diethylhydroxylamin, Imine, wie z. B. Methylethylimin oder Methylenviolett, Sulfonamide, wie z. B. N-Methyl-4-toluolsulfonamid oder N-tert.-Butyl-4-toluolsulfonamid, Oxime, wie Aldoxime, Ketoxime oder Amidoxime, wie z. B. Diethylketoxim, Methylethylketoxim oder Salicyladoxim, phosphorhaltige Verbindungen, wie z. B. Triphenylphosphin, Triphenylphosphit, Triethylphosphit, hypophosphorige Säure oder Alkylester der phosphorigen Säuren; schwefelhaltige Verbindungen wie z. B. Diphenylsulfid oder Phenothiazin; Metallsalze, wie Kupfer- oder Mangan-, Cer-, Nickel-, Chromsalze, beispielsweise -chloride, -sulfate, -salicylate, -tosylate, -acrylate oder -acetate, wie z. B. Kupferacetat, Kupfer(II)chlorid, Kupfersalicylat, Cer(III)acetat oder Cer(III)ethylhexanoat, oder Gemische davon sein.

Bevorzugt wird als Polymerisationsinhibitor bzw. Polymerisationsinhibitor-Gemisch mindestens eine Verbindung aus der Gruppe Hydrochinon, Hydrochinonmonomethylether, Phenothiazin, 4-Hydroxy-2,2,6,6-tetramethylpiperidin-N-oxyl, 4-Oxo-2,2,6,6-tetramethylpiperidin-N-oxyl, Bis(1-oxyl-2,2,6,6-tetramethyl-piperidine-4-yl)sebacat, 2-tert.-Butyl-phenol, 4-tert.-Butylphenol, 2,4-Di-tert.-Butylphenol, 2-tert.-Butyl-4-methylphenol, 6-tert.-Butyl-2,4-dimethylphenol, 2,6-Di-tert.-Butyl-4-methylphenol, 2-Methyl-4-tert.-butylphenol, hypophosphorige Säure, Kupfer(II)acetat, Kupfer(I)chlorid, Kupfer(II)chlorid, Kupfer(II)salicylat und Cer(III)acetat eingesetzt.

Besonders bevorzugt wird Phenothiazin (PTZ) und/oder Hydrochinonmonomethylether (MEHQ) als Polymerisationsinhibitor verwendet.

Ganz besonders bevorzugt wird PTZ als Polymerisationsinhibitor bei der Veresterung oder bei einer optionalen Verwendung einer Azeotrop-Rektifikationskolonneneinheit (25) eingesetzt. Im Fall der optional, nachgeschalteten Verfahrensschritte wird bei der Cyclohexan-Abtrennung IV und/oder bei der 2-Octanol-Abtrennung V auch ganz besonders bevorzugt PTZ verwendet.

Ganz besonders wird MEHQ als Polymerisationsinhibitor bei den optional, nachgeschalteten Verfahrensschritten wird bei der Reinsieder-Abtrennung VI und/oder der Schwersieder-Abtrennung VII verwendet.

Bevorzugt wird der Polymerisationsinhibitor in einer oder mehreren flüssigen, organischen Verbindungen gelöst. Die organische Verbindung ist bevorzugt 2-Octanol und/oder 2-Octyl(meth)acrylat.

Als Veresterungskatalysator kommen die üblichen Mineralsäuren und Sulfonsäuren in Frage, vorzugsweise Schwefelsäure, Phosphorsäure, Alkylsulfonsäuren (z. B. Methansulfonsäure, Trifluormethansulfonsäure) und Arylsulfonsäuren (z. B. Benzol-, p-Toluol- oder Dodecylbenzolsulfonsäure) oder Gemische davon, aber auch saure Ionenaustauscher oder Zeolithe sind einsetzbar. Besonders bevorzugt ist Schwefelsäure, Methansulfonsäure, p-Toluolsulfonsäure, m-Toluolsulfonsäure, o-Toluolsulfonsäure oder Gemische davon.

Ganz besonders bevorzugt wird Methansulfonsäure als Veresterungskatalysator verwendet.

In dem erfindungsgemäßen Verfahren werden die Edukte (Meth)acrylsäure und 2-Octanol sowie das Schleppmittel Cyclohexan kontinuierlich oder diskontinuierlich dem einen Reaktor (1) der Reaktoreinheit (24) zugeführt, wobei die kontinuierliche Zuführung bevorzugt wird.

In dem erfindungsgemäßen Verfahren wird als Schleppmittel Cyclohexan eingesetzt. Dieses besitzt bei Normaldruck eine Siedepunkt von 81 °C. Das Cyclohexan bildet mit dem Veresterungswasser ein heterogenes Azeotrop und dieses heterogene Azeotrop weist bei Normaldruck eine Siedetemperatur von nur 70 °C auf. Das heterogene Azeotrop hat mit seiner Siedetemperatur von 70 °C eine niedrigere Siedetemperatur als reines Wasser oder als andere Azeotrope wie beispielsweise 2-Octanol mit Veresterungswasser, Toluol mit Veresterungswasser oder Octene mit Veresterungswasser. Somit wird unter Verwendung von Cyclohexan weniger Energie benötigt, um das heterogene Azeotrop zu verdampfen.

Es wurde erkannt, dass man durch die Zuführung einer optimalen Menge an Schleppmittel Cyclohexan in den einen Reaktor (1) der Reaktoreinheit (24) eine geringere Sumpftemperatur einstellen kann als bei einem anderen Schleppmittel oder bei einer nicht-optimalen Menge an Cyclohexan, so dass sich weniger Nebenkomponenten bilden und zugleich der Gasstrom neben dem gasförmigen heterogenen Azeotrop möglichst rein ist, und dieser Gasstrom somit wenig andere Komponenten, wie beispielhaft (Meth)acrylsäure, enthält. Somit kann die zur Verdampfung des Veresterungswassers erforderliche Sumpftemperatur auch signifikant durch die Menge des eingesetzten Schleppmittels beeinflusst werden.

Eine niedrigere Sumpftemperatur reduziert unter anderem die Bildung von Nebenkomponenten wie zum Beispiel Octene, insbesondere 1-Octen sowie 2-Octen. Octene selbst wirken auch als Schleppmittel, haben aber einen deutlich höheren Siedepunkt als 2-Octanol. So hat beispielhaft 1-Octen bei Normaldruck eine Siedetemperatur von 121 °C. Die gebildeten Nebenkomponenten erhöhen auch das Trennproblem, das heterogene Azeotrop von dem Reaktionsgemisch abzutrennen sowie in der Reindestillation.

Überraschenderweise wurde gefunden, dass sich bei Verwendung gleicher Schleppmittelkonzentrationen und gleicher sonstiger Bedingungen, wie die Temperatur, Reaktionszeit, molare Verhältnisse der Einsatzstoffe, beim Einsatz von Cyclohexan der Umsatz höher ist, aber sehr viel weniger Nebenkomponenten gebildet werden, obwohl sich die Sumpftemperatur nicht signifikant unterscheidet und die Sumpftemperatur am Ende des Verfahrens sogar bei Verwendung von Cyclohexan am höchsten ist, bezogen auf die anderen Schleppmittel Toluol und 2-Octanol im Überschuss oder keinem Schleppmittel.

Ohne Schleppmittel verbleibt sehr viel Wasser im Sumpf. Um das Wasser effektiv zu entfernen, müsste das Verfahren unter Vakuumbedingungen betrieben werden. Jedoch sind Verfahren unter Vakuumbedingungen sehr aufwendig und verursachen hohe Kosten im Betrieb und bei der Errichtung der Anlage für das Verfahren.

Die niedrigere Siedetemperatur des heterogenen Azeotrops in dem einen Reaktor (1) bewirkt auch, dass weniger (Meth)acrylsäure in dem einen Reaktor (1) und/oder dem Reaktorheizelement (30) verdampft bzw. verdunstet. Dadurch erhöht sich die Trennleistung, das heterogene Azeotrop von dem Reaktionsgemisch abzutrennen.

In dieser Schrift definiert das "Reaktionsgemisch" ein flüssiges Gemisch, welches bei der Veresterung in dem einen Reaktor (1) gebildet wird und somit auch das Wertprodukt 2-Octyl(meth)acrylat enthält.

In dieser Schrift umfasst das "Startgemisch" die Edukte 2-Octanol und (Meth)acrylsäure sowie zusätzlich den sauren Veresterungskatalysator, den/die Polymerisationsinhibitoren und das Schleppmittel Cyclohexan. Durch die Veresterung geht das Startgemisch in das Reaktionsgemisch über.

In dieser Schrift umfasst die Sumpftemperatur einen Temperaturbereich von 90 bis 130 °C. Durch die Veränderung der Sumpftemperatur in diesem Bereich kann auch die Reaktionskinetik, die Ausbeute / der Umsatz, die Selektivität und/oder die Bildung von Nebenkomponenten usw. beeinflusst werden.

In dieser Schrift wird unter den Begriffen "Reaktorsumpf" oder "Sumpf des Reaktors" ein flüssiges Gemisch verstanden, welches sich unterhalb einer Gasgemisch-Phase im Reaktor (1) befindet. Somit ist der Reaktor (1) nur teilweise mit einem flüssigen Gemisch befüllt, da bei der Veresterung auch ein Teil aus dem flüssigen Gemisch, bevorzugt das heterogene Azeotrop, verdampft und dadurch den Reaktorraum oberhalb des flüssigen Gemischs befüllt.

In dieser Schrift definiert der Begriff "Rektifikationskolonneneinheit" eine oder mehrere Rektifikationskolonnen, die auch weitere Standardbauteile wie beispielhaft Druckminderer, Durchflussregler oder Sensoren enthalten können. Die Rektifikationskolonneneinheit umfasst somit auch ihre Regelung. Im Fall von mehreren Rektifikationskolonnen können diese miteinander per Reihen- oder Parallelschaltung verbunden sein.

Rektifikationskolonnen sind von an sich bekannter Bauart und weisen die üblichen Apparate wie beispielsweise einen Verdampfer im Sumpf, einen Verdampfer im Schwersieder-Ablauf oder einen Kondensator im Leichtsieder-Ablauf auf, wobei die Schwersieder sich bevorzugt im Sumpfbereich und die Leichtsieder bevorzugt im Kopfbereich der Rektifikationskolonne befinden. Typischerweise wird ein Teil des Massenstroms des Schwersieder-Ablaufs in den Sumpfbereich der Rektifikationskolonne zurückgeführt. Prinzipiell ist es aber auch möglich, dass der Sumpfbereich über beispielsweise eine Außenwandbeheizung der Kolonne im Sumpfbereich beheizt wird und/oder ein Verdampfer im Sumpfbereich integriert ist.

Typischerweise wird der Massenstrom des Leichtsieder-Ablaufs nach dem Kondensieren in einem Kondensator im Bereich von 10 bis 200 Gew.-% in den Kopfbereich der Rektifikationskolonne zurückgeführt.

Als Kolonneneinbauten kommen prinzipiell alle gängigen Einbauten in Betracht, beispielsweise Böden, Packungen und/oder Schüttungen. Von den Böden sind Glockenböden, Siebböden, Ventilböden, Thormannböden und/ oder Dual-Flow-Böden bevorzugt, von den Schüttungen sind solche mit Ringen, Wendeln, Sattelkörpern oder Geflechten bevorzugt.

In dieser Schrift werden Stoffkomponenten als Leichtsieder bezeichnet, wenn bei Normaldruck die Siedetemperatur kleiner als die Siedetemperatur von 2-Octylacrylat ist. Analog werden Stoff-komponenten als Schwersieder bezeichnet, wenn bei Normaldruck die Siedetemperatur größer als oder gleich der Siedetemperatur von 2-Octylacrylat ist. Die Siedetemperatur von 2-Octylacrylat beträgt 211 °C bei Normaldruck.

In dieser Schrift definiert der Begriff "Reaktor mit einem Reaktorheizelement" allgemein einen Reaktor (1) innerhalb einer Reaktoreinheit (24), der ein oder mehrere Reaktorheizelement(e) (30) innerhalb und/oder außerhalb des Reaktors (1) aufweist, die das Reaktionsgemisch erhitzen. Das Reaktorheizelement (30) oder eines der Reaktorheizelemente ist beispielsweise ein Tauchsieder im Reaktor (1), ein auf die äußere Mantelfläche des Reaktors (1) und/oder ein innerhalb des Reaktors (1) angeordnetes Rohrsystem umfassend Rohrschlangen oder Halbrohrschlangen, ein auf die äußere Mantelfläche des Reaktors (1) und/oder ein innerhalb des Reaktors (1) angeordnetes elektrisches Heizsystem, ein sich außerhalb des Reaktors (1) befindlicher Verdampfer, wobei das Reaktionsgemisch zumindest teilweise durch den Verdampfer strömt oder eine doppelwandige Ausführungsform der Reaktoraußenwand, in der eine von dem Reaktionsgemisch separiertes Fluid, wie eine Flüssigkeit, ein Gas und/oder ein Heizdampf, temperiert wird und dadurch eine vorbestimmte Heiztemperatur eingestellt wird, wodurch sich das Reaktionsgemisch im Reaktor (1) erhitzt. Generell können mehrere Reaktorheizelemente verwendet werden, um das Reaktionsgemisch im Reaktor (1) zu erhitzen. So kann beispielhaft eine doppelwandige Ausführungsform der Reaktoraußenwand und ein sich außerhalb des Reaktor (1) befindlichen Verdampfers zeitgleich oder zumindest teilweise zeitlich versetzt das Reaktionsgemisch erhitzen.

In dieser Schrift wird unter dem Begriff "Verdampfer" auch ein Reaktorheizelement (30) verstanden. Der Verdampfer kann auch weitere Standardbauteile enthalten, wie beispielhaft Regelventile, Druckminderer, Durchflussregler oder Sensoren. Der Verdampfer kann somit auch eine Regelung umfassen. Generell können unter dem Begriff "Verdampfer" auch mehreren Verdampfern verstanden werden, welche miteinander per Reihen- oder Parallelschaltung verbunden sind.

Beispiele für geeignete Verdampfer sind Dünnschicht-, Fallfilm-, Naturumlauf- und Zwangsumlaufverdampfer. Die Verdampfer können als Rohrbündelwärmeübertrager oder Plattenwärmeübertrager ausgeführt werden. Dem Fachmann sind geeignete Verdampfer bekannt und unter anderem beschrieben in: SPX, Evaporator Handbook, APV, verfügbar unter https://user-pages.umbc.edu/~dfrey1/ench445/apv evap.pdf (aufgerufen am 1.11.2021).

In dieser Schrift definiert der Begriff "Reaktoreinheit" einen oder mehrere Reaktoren, die auch weitere Standardbauteile wie beispielhaft Druckminderer, Durchflussregler, Reaktorheizelemente, weitere Heizelemente, Leitungen, Wärmeübertrager oder Verdampfer enthalten können. Die Reaktoreinheit (24) kann auch Regelventile und/oder Sensoren umfassen. Somit kann die Reaktoreinheit (24) auch eine Prozessregelung enthalten. Selbstverständlich können auch mehrere Wärmeübertrager pro Reaktor oder mehrere Wärmeübertrager im Verbund für alle Reaktoren der Reaktoreinheit (24) vorhanden sein.

In einer weiteren, bevorzugten Ausgestaltung sind im Fall mehrerer Reaktoren die Reaktoren miteinander parallel verschaltet.

In einer bevorzugten Ausgestaltung umfasst die Reaktoreinheit (24) mehrere Reaktoren, wobei die Reaktoren miteinander in Reihe verschaltet. Somit kann eine Kaskadierung von zwei oder mehreren hintereinander geschalteten Reaktoren ermöglicht werden, wobei mit der Kaskadierung der Austragsstrom eines Reaktors den Zulaufstrom des nächstfolgenden Reaktors bildet.

In einer bevorzugten Ausgestaltung der Kaskadierung werden die Komponenten für die Veresterung nur einem ersten Reaktor (1) zugeführt und nur der durch die Veresterung resultierende Reaktionsaustrag wird jeweils dem nachfolgenden Reaktor zugeführt.

In einer bevorzugten Ausgestaltung ist der eine Reaktor (1) mit innen- oder außenliegenden Heizschlangen und/oder mit einer doppelwandigen Ausführungsform der Reaktoraußenwand ausgestattet.

In einer bevorzugten Ausgestaltung ist der eine Reaktor (1) mit einem außenliegenden Verdampfer, mit innen- und/oder außenliegenden Heizschlangen und/oder mit einer doppelwandigen Ausführungsform der Reaktoraußenwand ausgestattet.

In einer bevorzugten Ausgestaltung ist der eine Reaktor (1) mit einem außenliegenden Verdampfer und mit einer doppelwandigen Ausführungsform der Reaktoraußenwand ausgestattet.

In einer bevorzugten Ausgestaltung ist der eine Reaktor (1) mit einem außenliegenden Verdampfer und mit innen- und/oder außenliegenden Heizschlangen ausgestattet.

In einer bevorzugten Ausgestaltung enthält der eine Reaktor (1) einen außenliegenden Wärmeübertrager und/oder Verdampfer.

In einer bevorzugten Ausführungsform enthält der eine Reaktor (1) einen Verdampfer. Im Fall von mehreren Reaktoren enthält jeder einzelne Reaktor einen eigenen Verdampfer.

In einer weiteren Ausführungsform enthält die Reaktoreinheit (24) einen Verdampfer, mit dem alle Reaktoren der Reaktoreinheit (24) verbunden sind.

In einer bevorzugten Ausführungsform wird der eine Reaktor (1) im Naturumlauf betrieben. Dies hat den Vorteil, dass der Reaktorapparat günstiger zu beziehen ist, weil er unter anderem kein Rührwerk oder andere mechanische Hilfsmittel benötigt. Das Schleppmittel Cyclohexan sorgt für eine erhöhte Durchmischung.

In einer bevorzugten Ausführungsform wird die Veresterung kontinuierlich betrieben, bei der die Zuführung der Edukte und des Schleppmittels Cyclohexan kontinuierlich erfolgt und die Verdampfereinheit auch kontinuierlich betrieben wird.

In einer weiteren Ausführungsform wird die Veresterung diskontinuierlich betrieben, bei der die Zuführung der Edukte und des Schleppmittels Cyclohexan diskontinuierlich erfolgt.

In einer bevorzugten Ausführungsform des Verfahrens wird das gasförmige heterogene Azeotrop eine der Reaktoreinheit (24) nachgeschalteten Azeotrop-Rektifikationskolonneneinheit (25) zugeführt, wobei sich eine Azeotrop-Rektifikationskolonne (4) innerhalb der Azeotrop-Rektifikationskolonneneinheit (25) befindet, und die eine Azeotrop-Rektifikationskolonne (4) bei einem Absolutdruck im Bereich von 0,5 bis 2,0 bar und bei einer Sumpftemperatur im Bereich von 90 bis 130 °C betrieben wird, und das heterogene Azeotrop über den Kopf der einen Azeotrop-Rektifikationskolonne (4) abgetrennt, in einem Kondensator kondensiert und anschließend einem Phasenscheider (6) zugeführt wird, wobei im Phasenscheider (6) das Veresterungswasser als untere Phase abgeschieden wird, wohingegen das Cyclohexan als obere Phase im Phasenscheider (6) abgetrennt wird.

In einer weiteren Ausgestaltung enthält im Fall mehrerer Reaktoren jeder einzelne Reaktor eine auf ihn aufgesetzte Azeotrop-Rektifikationskolonneneinheit (25).

In einer weiteren Ausgestaltung enthält im Fall mehrerer Reaktoren jeder einzelne Reaktor eine auf ihn aufgesetzte Azeotrop-Rektifikationskolonneneinheit (25), wobei die Azeotrop-Rektifikationskolonneneinheit (25) bevorzugt genau eine Azeotrop-Rektifikationskolonne (4) enthält.

In einer besonders bevorzugten Ausgestaltung wird eine Azeotrop-Rektifikationskolonneneinheit (25) auf die gesamte Reaktoreinheit (24) aufgesetzt, wobei die Azeotrop-Rektifikationskolonneneinheit (25) bevorzugt genau eine Azeotrop-Rektifikationskolonne (4) enthält. Dies hat den Vorteil, dass nur eine Azeotrop-Rektifikationskolonne (4) für den oder alle Reaktor(en) betrieben werden muss und somit das Verfahren energie- und kosteneffizient ist.

In einer bevorzugten Ausführungsform mit mehreren Reaktoren werden die aufsteigenden Dämpfe aus allen Reaktoren einer einzigen Azeotrop-Rektifikationskolonne (4) zugeführt, wobei der flüssige Ablauf der Azeotrop-Rektifikationskolonne (4) nur in den ersten Reaktor (1) zurückgeführt wird. Die Azeotrop-Rektifikationskolonneneinheit (25) enthält vorzugsweise nur eine Azeotrop-Rektifikationskolonne.

In einer bevorzugten Ausführungsform wird die Azeotrop-Rektifikationskolonneneinheit (25) kontinuierlich betrieben.

In einer bevorzugten Ausführungsform wird das im Phasenscheider (6) als obere Phase gewonnene Cyclohexan zumindest teilweise oder vollständig der Reaktoreinheit (24) zurückgeführt.

In einer bevorzugten Ausgestaltung wird das im Phasenscheider (6) als obere Phase gewonnene Cyclohexan zu einem Gewichtsanteil im Bereich von 40 bis 100 Gew.-%, bevorzugt im Bereich von 50 bis 99,9 Gew.-%, der Reaktoreinheit (24) zurückgeführt, und der sich ergebende Restanteil des gewonnenen Cyclohexans im Bereich von unterhalb des Kopfs bis zur Mitte der einen Azeotrop-Rektifikationskolonne (4) zurückgeführt.

Dies hat den Vorteil, dass der Schleppmittelanteil in der Reaktoreinheit (24) und/oder der Azeotrop-Rektifikationskolonneneinheit (25) eingestellt werden kann, um das Veresterungswasser effizient abtrennen zu können oder auch um eine gezielte Einstellung der Sumpftemperatur durch Zufuhr von Cyclohexan in den Reaktor (1) durchführen zu können.

In einer bevorzugten Ausführungsform wird der Phasenscheider (6) kontinuierlich betrieben, indem die Zu- und Abläufe kontinuierlich strömen.

Verfahrensstufe II: Alkalische Extraktion

In einer bevorzugten Ausgestaltung erfolgt im Anschluss an die Veresterung eine alkalische Extraktion des resultierenden Reaktionsaustrags mittels einer alkalischen Lösung, indem u.a. der saure Veresterungskatalysator sowie nicht umgesetzte (Meth)acrylsäure in einer Neutralisations-Extraktionseinheit (7) neutralisiert werden, und sich dadurch eine obere Neutralisations-Phase, welche 2-Octyl(meth)acrylat enthält, und eine untere Neutralisations-Phase, welche durch die Neutralisation erzeugte Salze sowie Wasser enthält, ergeben und diese beiden Phasen somit voneinander getrennt vorliegen. Bevorzugt werden die beiden Neutralisations-Phasen durch ein Dispergierapparat, wie z.B. eine Mischpumpe, erzeugt und anschließend in einem Phasenscheider (6), der sich innerhalb der Neutralisations-Extraktionseinheit (7) befindet, voneinander getrennt.

In einer bevorzugten Ausführungsform wird die Neutralisations-Extraktionseinheit (7) kontinuierlich betrieben, indem die Zu- und Abläufe kontinuierlich strömen.

Verfahrensstufe III: Wasserwäsche Extraktion

In einer bevorzugten Ausgestaltung erfolgt im Anschluss an die alkalische Extraktion des Reaktionsaustrags eine Extraktion der aus der Neutralisations-Extraktionseinheit (7) gewonnenen oberen Neutralisations-Phase mit Wasser in einer Wasserwäsche-Extraktionseinheit (8), wobei eine Wasser und Reste von Salzen enthaltende untere Wasserwäsche-Phase und eine 2-Octyl(meth)acrylat enthaltende obere Wasserwäsche-Phase gebildet werden und diese beiden Phasen somit voneinander getrennt vorliegen. Bevorzugt werden die beiden Wasserwäsche-Phasen durch ein Dispergierapparat, wie z.B. eine Mischpumpe, erzeugt und anschließend in einem Phasenscheider (6), der sich innerhalb der Wasserwäsche-Extraktionseinheit (8) befindet, voneinander getrennt.

Sowohl die alkalische Extraktion (Verfahrensstufe II) als auch die Wasserwäsche Extraktion (Verfahrensstufe III) können beispielsweise in einem Rührbehälter oder in einer anderen herkömmlichen Apparatur, z. B. in einer Kolonne oder in einer Mixer-Settler-Apparatur durchgeführt werden. Bei einem Mixer-Settler oder einer Kolonne werden die entsprechenden Verfahren bevorzugt kontinuierlich durchgeführt. Bei einem Rührbehälter werden die entsprechenden Verfahren bevorzugt diskontinuierlich durchgeführt.

Für die Mixer-Settler-Apparatur gibt es unterschiedliche Dispergiermöglichkeiten, wie z.B. die Dispergierung in einem Rührbehälter, in einem statischer Mischer, in einer Rohrleitung oder in einer Mischpumpe und einen Phasentrennapparat.

Mixer und Settler können mehr oder weniger unabhängig voneinander konstruiert werden. Der Mischvorgang kann beispielsweise durch die richtige Wahl des Rührers, der Rührgeschwindigkeit, des Leistungseintrags und/oder des Durchsatzes angepasst werden. Im Fall einer Mischpumpe kann bevorzugt der Leistungseintrag angepasst werden.

Sowohl die alkalische Extraktion (Verfahrensstufe II) als auch die Wasserwäsche Extraktion (Verfahrensstufe III) können bevorzugt eine Extraktionskolonne, ein Zentrifugalextraktor oder eine Mixer-Settler-Apparatur sein.

In einer besonders bevorzugten Ausführungsform wird im Fall einer alkalischen Extraktion (Verfahrensstufe II) der resultierende Reaktionsaustrag durch eine Pumpe, wie z.B. eine Mischpumpe, mit einer verdünnten Lauge dispergiert und dann einem Phasenscheider (6) zugeführt, der die obere und untere Neutralisations-Phase voneinander trennt.

Verfahrenstechnisch können alle an sich bekannten Extraktions- und Waschverfahren und Extraktionsapparate eingesetzt werden, z. B. solche, die in Ullmann's Encyclopedia of Industrial Chemistry, 6th edition, 1999 Electronic Release, Kapitel: Liquid - Liquid Extraction - Apparatus, beschrieben sind. Beispielsweise können dies ein- oder mehrstufige Extraktionen, bevorzugt einstufige Extraktionen, sein, die in Gleich- oder Gegenstromfahrweise betrieben werden können.

Die Wasserwäsche wird bevorzugt dann eingesetzt, wenn Salze abgetrennt werden sollen.

In einer bevorzugten Ausführungsform wird die Wasserwäsche-Extraktionseinheit (8) kontinuierlich betrieben, indem die Zu- und Abläufe kontinuierlich strömen.

In einer besonders bevorzugten Ausführungsform wird ein statischer Mischer verwendet, der die aus der Neutralisations-Extraktionseinheit (7) gewonnene obere Neutralisations-Phase mit separat zugeführtem Wasser dispergiert. Anschließend wird das aus dem statischen Mischer dispergierte Gemisch einem Phasenscheider (6) zugeführt, in dem sich eine mit Wasser und Reste von Salzen enthaltende untere Wasserwäsche-Phase und eine mit 2-Octyl(meth) acrylat enthaltende obere Wasserwäsche-Phase ausbildet.

Die folgenden Verfahrensstufen IV bis VII werden bevorzugt kontinuierlich betrieben.

Verfahrensstufe IV: Cyclohexan-Abtrennung

In einer bevorzugten Ausgestaltung erfolgt im Anschluss an die alkalische Extraktion des Reaktionsaustrags eine Abtrennung des Schleppmittels Cyclohexan aus der oberen Neutralisations-Phase in einer Schleppmittel-Rektifikationskolonneneinheit, wobei sich eine Schleppmittel-Rektifikationskolonne (9) innerhalb der Schleppmittel-Rektifikationskolonneneinheit befindet, und die eine Schleppmittel-Rektifikationskolonne (9) bei einem verminderten Absolutdruck im Bereich von 0,05 bis 0,9 bar und bei einer Sumpftemperatur im Bereich von 70 bis 120 °C betrieben wird, und über den Kopf der einen Schleppmittel-Rektifikationskolonne (9) eine organische Phase abgezogen wird, die das Schleppmittel Cyclohexan, Octene, 2-Octanol sowie einen Massenstromanteil an 2-Octyl(meth)acrylat im Bereich von 0,1 bis 5,0 %, bezogen auf die zugeführte obere Neutralisations-Phase, enthält, wobei die über Kopf abgeführten Komponenten einen Schleppmittel-Massenstrom bilden, dieser kondensiert wird und im Bereich von 50 bis 100 % dem einen Reaktor (1) der Reaktoreinheit (24) zugeführt wird, wobei der restliche Schleppmittel-Massenstromanteil kondensiert und am Kopf der Schleppmittel-Rektifikationskolonne (9) zurückgeführt wird, wohingegen Schwersieder, welche Di(meth)acrylsäureester und Oxiester, wie den Alkoxialkylester der (Meth)acrylsäure, enthalten, sowie 2-Octanol und 2-Octyl(meth)acrylat durch einen Sumpfablauf der einen Schleppmittel-Rektifikationskolonne (9) abgezogen werden, wobei im Bereich von 20 bis 95 % des Massenstroms des Sumpfablaufs durch einen Verdampfer strömt und anschließend in die eine Schleppmittel-Rektifikationskolonne (9) zurückgeführt wird.

In einer bevorzugten Ausgestaltung erfolgt im Anschluss an die Wasserwäsche-Extraktion eine Abtrennung des Schleppmittels Cyclohexan aus der oberen Wasserwäsche-Phase in einer Schleppmittel-Rektifikationskolonneneinheit, wobei sich eine Schleppmittel-Rektifikationskolonne (9) innerhalb der Schleppmittel-Rektifikationskolonneneinheit befindet, und die eine Schleppmittel-Rektifikationskolonne (9) bei einem verminderten Absolutdruck im Bereich von 0,05 bis 0,9 bar und bei einer Sumpftemperatur im Bereich von 70 bis 120 °C betrieben wird, und über den Kopf der einen Schleppmittel-Rektifikationskolonne (9) eine organische Phase abgezogen wird, die das Schleppmittel Cyclohexan, Octene, 2-Octanol sowie einen Massenstromanteil an 2-Octyl(meth)acrylat im Bereich von 0,1 bis 5,0 %, bezogen auf die zugeführte obere Wasserwäsche-Phase, enthält, wobei die über Kopf abgeführten Komponenten einen Schleppmittel-Massenstrom bilden, dieser kondensiert wird und im Bereich von 50 bis 100 Gew.-% dem einen Reaktor (1) der Reaktoreinheit (24) zugeführt wird, wobei der restliche Schleppmittel-Massenstromanteil kondensiert und am Kopf der Schleppmittel-Rektifikationskolonne (9) zurückgeführt wird, wohingegen Schwersieder, welche Di(meth)acrylsäureester und Oxiester, wie den Alkoxialkylester der (Meth)acrylsäure, enthalten, sowie 2-Octanol und 2-Octyl(meth)acrylat durch einen Sumpfablauf der einen Schleppmittel-Rektifikationskolonne (9) abgezogen werden, wobei im Bereich von 20 bis 95 % des Massenstroms des Sumpfablaufs durch einen Verdampfer strömt und anschließend in die eine Schleppmittel-Rektifikationskolonne (9) zurückgeführt wird.

In einer besonders bevorzugten Ausgestaltung befindet sich genau eine Schleppmittel-Rektifikationskolonne (9) innerhalb der Schleppmittel-Rektifikationskolonneneinheit.

Verfahrensstufe V: 2-Octanol-Abtrennung

In einer besonders bevorzugten Ausgestaltung erfolgt im Anschluss an die Abtrennung des Schleppmittels Cyclohexan eine Abtrennung des 2-Octanols aus dem durch den Sumpfablauf der einen Schleppmittel-Rektifikationskolonne (9) gegebenen Massenstrom in einer 2-Octanol-Rektifikationskolonneneinheit, wobei sich eine 2-Octanol-Rektifikationskolonne (10) innerhalb der 2-Octanol-Rektifikationskolonneneinheit befindet, und die eine

2-Octanol-Rektifikationskolonne (10) bei einem verminderten Absolutdruck im Bereich von 0,005 bis 0,10 bar und bei einer Sumpftemperatur im Bereich von 70 bis 130 °C betrieben wird, und der durch den Sumpfablauf der einen Schleppmittel-Rektifikationskolonne (9) gegebene Massenstrom im Bereich von unterhalb des Kopfs bis zur Mitte der 2-Octanol-Rektifikationskolonne (10) zudosiert wird, und die über den Kopf der einen 2-Octanol-Rektifikationskolonne (10) abgeführten Komponenten kondensiert werden und im Bereich von 20 bis 50 %, bezogen auf den Massenstrom der über Kopf abgeführten Komponenten, dem einen Reaktor (1) der Reaktoreinheit (24) zugeführt werden, wobei die abgeführten Komponenten im Bereich von 2 bis 40 Gew.-% aus 2-Octanol bestehen. Andererseits werden 2-Octyl(meth)acrylat und Schwersieder, die Di(meth)acrylsäureester und Oxiester enthalten, durch einen Sumpfablauf der 2-Octanol-Rektifikationskolonne (10) abgezogen, wobei 20 bis 95 % des Massenstroms des Sumpfablaufs durch einen Verdampfer strömt und anschließend in die eine 2-Octanol-Rektifikationskolonne (10) zurückgeführt wird.

In einer bevorzugten Ausgestaltung erfolgt im Anschluss an die Abtrennung des Schleppmittels Cyclohexan eine Abtrennung des 2-Octanols aus dem durch den Sumpfablauf der einen Schleppmittel-Rektifikationskolonne (9) gegebenen Massenstrom in einer 2-Octanol Verdampfereinheit, wobei sich ein 2-Octanol-Verdampfer (110) innerhalb der 2-Octanol-Verdampfereinheit befindet, und der eine 2-Octanol-Verdampfer (110) bei einem verminderten Absolutdruck im Bereich von 0,005 bis 0,10 bar und bei einer Sumpftemperatur im Bereich von 70 bis 130 °C betrieben wird, und der durch den Sumpfablauf der einen Schleppmittel-Rektifikationskolonne (9) gegebene Massenstrom an einem 2-Octanol Einlass des einen 2-Octanol-Verdampfers zudosiert wird, und die über einen 2-Octanol-Verdampfer (110) Auslass des einen 2-Octanol-Verdampfers abgeführten Komponenten im Bereich von 20 bis 50 %, bezogen auf den Massenstrom der am 2-Octanol-Verdampfer Auslass abgeführten Komponenten, der Reaktoreinheit (24) zugeführt werden, wobei diese abgeführten Komponenten im Bereich von 2 bis 40 Gew.-% aus 2-Octanol bestehen. Andererseits werden 2-Octyl(meth)acrylat und Schwersieder, die Di(meth)acrylsäureester und Oxiester enthalten, durch einen Sumpfablauf des einen 2-Octanol-Verdampfers abgezogen, wobei im Bereich von 20 bis 95 % des Massenstroms des Sumpfablaufs durch einen Verdampfer strömt und anschließend in den einen 2-Octanol-Verdampfer (110) zurückgeführt wird.

In einer bevorzugten Ausgestaltung wird nach der Cyclohexan-Abtrennung das 2-Octanol und zugleich das 2-Octyl(meth)acrylat in einer Trennwandkolonne (131) von den Cyclohexan enthaltenden restlichen Komponenten abgetrennt.

In einer bevorzugten Ausgestaltung ist die Trennwandkolonne (131) mit einer senkrechten Trennwand (138) versehen, die in Teilbereichen eine Quervermischung von Flüssigkeits- und Dampfströmen verhindert. Die Trennwand (138), die aus einem ebenen Blech bestehen kann, unterteilt die Kolonne in Längsrichtung in deren mittlerem Bereich in einen Zulaufteil (140) und einen Entnahmeteil (141).

In einer bevorzugten Ausgestaltung wird das aufzutrennende Gemisch, das Leichtsieder, 2-Octyl(meth)acrylat und Schwersieder enthält, dem Zulaufteil (140) zugeführt und 2-Octyl(meth)acrylat wird aus dem Entnahmeteil (141) dampfförmig oder flüssig abgezogen. Die Leichtsieder werden über Kopf der Trennwandkolonne (131) und die Schwersieder über den Sumpf der Trennwandkolonne (131) abgetrennt.

Der Energiebedarf und die Investitionskosten liegen um ca. 25 % günstiger als bei einer konventionellen Kolonnenverschaltung mit zwei Rektifikationskolonnen.

Verfahrensstufe VI: Reinsieder-Abtrennung (Abtrennung des Zielprodukts 2-Octyl(meth)acrylat)

In einer bevorzugten Ausgestaltung erfolgt im Anschluss an die Abtrennung des 2-Octanols eine Reinsieder-Abtrennung aus dem durch den Sumpfablauf der einen 2-Octanol-Rektifikationskolonne (10) gegebenen Massenstrom in einer Reinsieder-Rektifikationskolonneneinheit, wobei sich eine Reinsieder-Rektifikationskolonne (11) innerhalb der Reinsieder-Rektifikationskolonneneinheit befindet, und die eine Reinsieder-Rektifikationskolonne (11) bei einem verminderten Absolutdruck im Bereich von 0,002 bis 0,05 bar und bei einer Sumpftemperatur im Bereich von 80 bis 130 °C betrieben wird, und der durch den Sumpfablauf der einen 2-Octanol-Rektifikationskolonne (10) gegebene Massenstrom im Bereich von unterhalb des Kopfs bis zur Mitte der einen Reinsieder-Rektifikationskolonne (11) zudosiert wird, und zur Stabilisierung der einen Reinsieder-Rektifikationskolonne (11) 2-Octyl(meth)acrylat sowie einen Polymerisationsinhibitor jeweils im Bereich von 0,01 bis 1,0 %, bezogen auf den durch den Sumpfablauf der einen 2-Octanol-Rektifikationskolonne (10) gegebenen Massenstrom, im Bereich vom Sumpf bis zur Mitte der einen Reinsieder-Rektifikationskolonne (11) zudosiert werden, und das 2-Octyl(meth)acrylat über den Kopf der einen Reinsieder-Rektifikationskolonne (11) abgezogen wird, wohingegen Schwersieder, welche Di(meth)acrylsäureester und Oxiester, wie den Alkoxialkylester der (Meth)acrylsäure, enthalten, durch einen Sumpfablauf der einen Reinsieder-Rektifikationskolonne (11) abgezogen werden.

In einer besonders bevorzugten Ausgestaltung erfolgt im Anschluss an die Abtrennung des 2-Octanols eine Reinsieder-Abtrennung aus dem durch den Sumpfablauf des einen 2-Octanol-Verdampfers gegebenen Massenstrom in einer Reinsieder-Verdampfereinheit, wobei sich ein Reinsieder-Verdampfer innerhalb der Reinsieder-Verdampfereinheit befindet, und der eine Reinsieder-Verdampfer (111) bei einem verminderten Absolutdruck im Bereich von 0,002 bis 0,05 bar und bei einer Sumpftemperatur im Bereich von 80 bis 130 °C betrieben wird, und der durch den Sumpfablauf der einen 2-Octanol-Rektifikationskolonne (10) oder des einen 2-Octanol-Verdampfers gegebene Massenstrom an einem Reinsieder-Verdampfer Einlass der einen Reinsieder-Verdampfereinheit zudosiert wird, und zur Stabilisierung des einen Reinsieder-Verdampfers 2-Octyl(meth)acrylat sowie einen Polymerisationsinhibitor jeweils im Bereich von 0,01 bis 1,0 %, bezogen auf den durch den Sumpfablauf des einen 2-Octanol-Verdampfers gegebenen Massenstrom, an einen Reinsieder-Verdampfer Einlass des einen Reinsieder-Verdampfers zudosiert werden, und das 2-Octyl(meth)acrylat über einen Reinsieder-Verdampfer Auslass des einen Reinsieder-Verdampfers abgezogen wird, wohingegen Schwersieder, welche Di(meth)acrylsäureester und Oxiester, wie den Alkoxialkylester der (Meth)acrylsäure, enthalten, durch einen Sumpfablauf des einen Reinsieder-Verdampfers abgezogen werden.

In einer ganz besonders bevorzugten Ausgestaltung enthält die Reinsieder-Verdampfereinheit genau einen Reinsieder-Verdampfer.

Verfahrensstufe VII: Schwersieder-Abtrennung (Di(meth)acrylsäureester und Oxiester, wie den Alkoxialkylester der (Meth)acrylsäure)

In einer bevorzugten Ausgestaltung erfolgt im Anschluss an die Reinsieder-Abtrennung eine Schwersieder-Abtrennung aus dem durch den Sumpfablauf der einen Reinsieder-Rektifikationskolonne (11) oder des einen Reinsieder-Verdampfers gegebenen Massenstrom in einer Schwersieder-Rektifikationskolonneneinheit, wobei sich eine Schwersieder-Rektifikationskolonne (12) innerhalb der Schwersieder-Rektifikationskolonneneinheit befindet, und die eine Schwersieder-Rektifikationskolonne (12) bei einem verminderten Absolutdruck im Bereich von 0,002 bis 0,05 bar und bei einer Sumpftemperatur im Bereich von 80 bis 150 °C betrieben wird, und der durch den Sumpfablauf der einen Reinsieder-Rektifikationskolonne (11) gegebene Massenstrom im Bereich vom Sumpf bis zur Mitte der einen Schwersieder-Rektifikationskolonne (12) zudosiert wird, und der über den Kopf der einen Schwersieder-Rektifikationskolonne (12) entnommenen Abzug kondensiert wird und im Bereich vom Sumpf bis zur Mitte der einen Reinsieder-Rektifikationskolonne (11) oder am Reinsieder-Verdampfer Einlass des einen Reinsieder-Verdampfers zugeführt wird.

In einer besonders bevorzugten Ausgestaltung erfolgt im Anschluss an die Reinsieder-Abtrennung eine Schwersieder-Abtrennung aus dem durch den Sumpfablauf der einen Reinsieder-Rektifikationskolonne (11) oder des einen Reinsieder-Verdampfers gegebenen Massenstrom in einer Schwersieder-Verdampfereinheit, wobei sich ein Schwersieder-Verdampfer innerhalb der Schwersieder-Verdampfereinheit befindet, und der eine Schwersieder-Verdampfer bei einem verminderten Absolutdruck im Bereich von 0,002 bis 0,05 bar und bei einer Sumpftemperatur im Bereich von 80 bis 150 °C betrieben wird, und der durch den Sumpfablauf des einen Reinsieder-Verdampfers gegebene Massenstrom an einen Schwersieder-Verdampfer Einlass des einen Schwersieder-Verdampfers zudosiert wird und dem einen Schwersieder-Verdampfer entnommenen Abzug kondensiert wird und im Bereich vom Sumpf bis zur Mitte der einen Reinsieder-Rektifikationskolonne (11) oder am Reinsieder-Verdampfer Einlass des einen Reinsieder-Verdampfers zurückgeführt wird.

In einer ganz besonders bevorzugten Ausgestaltung enthält die Schwersieder-Verdampfereinheit genau einen Schwersieder-Verdampfer.

Weitere bevorzugte Ausführungsformen für die Verfahrensstufen I und II

In einer bevorzugten Ausgestaltung beträgt die Sumpftemperatur in dem einen Reaktor (1) der Reaktoreinheit (24) im Bereich von 100 bis 125 °C, bevorzugt 110 bis 115 °C.

In einer bevorzugten Ausgestaltung findet die Veresterung bei einem Absolutdruck im Bereich von 0,8 bis 1,5 bar, besonders bevorzugt von 0,9 bis 1,2 bar, statt.

In einer bevorzugten Ausgestaltung werden im Sumpf des Reaktors (1) weniger als 1,0 % Octene, bezogen auf die Summe der dem Reaktor (1) zugeführten Massenströme 2-Octanol (13), sauren Veresterungskatalysator (15), Polymerisationsinhibitoren (31) und (Meth)acrylsäure (14), gebildet. Dies kann u.a. dadurch erreicht werden, wenn die Ausgestaltungen aus den beiden vorstehenden Absätzen angewendet werden.

In einer bevorzugten Ausgestaltung wird dem einen Reaktor (1) der Reaktoreinheit (24) Cyclohexan in einer Menge im Bereich von 100 bis 600 Gew.-%, bevorzugt im Bereich von 200 bis 500 Gew.-%, besonders bevorzugt im Bereich von 350 bis 450 Gew.-%, jeweils bezogen auf die Summe die dem einen Reaktor (1) der Reaktoreinheit (24) zugeführten Massenmengen an 2-Octanol (13) und (Meth)acrylsäure (14), zugeführt.

In einer bevorzugten Ausgestaltung wird dem einen Reaktor (1) der Reaktoreinheit (24) Cyclohexan mit einer Konzentration im Bereich von 10 bis 90 Gew.-% zusätzlich in den einen Reaktor (1) der Reaktoreinheit (24) zudosiert, wobei sich der Gewichtsprozentanteil der Konzentration auf die in dem einen Reaktor (1) der Reaktoreinheit (24) vorhandenen Komponenten inklusive dem zudosierten Cyclohexan bezieht.

In einer bevorzugten Ausgestaltung beträgt der Katalysator-Anteil in dem einen Reaktor (1) der Reaktoreinheit (24) maximal 10 Gew.-%, bezogen auf die Summe der in dem Reaktor (1) der Reaktoreinheit (24) vorhandenen Komponenten an 2-Octanol und (Meth)acrylsäure.

In einer bevorzugten Ausgestaltung ist die alkalische Lösung in der Verfahrensstufe (II) wässriges NaOH, welches im Bereich von 5 bis 25 Gew.-% NaOH, bevorzugt im Bereich von 10 bis 20 Gew.-% NaOH, enthält.

In einer bevorzugten Ausgestaltung wird in dem einen Reaktor (1) der Reaktorinhalt im Naturumlauf oder Zwangsumlauf umgewälzt.

In einer bevorzugten Ausgestaltung weisen die ohne das Schleppmittel Cyclohexan in den einen Reaktor (1) der Reaktoreinheit (24) insgesamt zufließenden Komponenten folgende Gewichtsanteile auf:

|   |   |   |   |   |
|---|---|---|---|---|
| • | 2-Octanol: | 40,00 | bis | 84,39 Gew.-% |
| • | (Meth)acrylsäure: | 15,00 | bis | 59,39 Gew.-% |
| • | saurer Veresterungskatalysator: | 0,50 | bis | 10,00 Gew.-% |

(fortgesetzt)

| | | | | |
|---|---|---|---|---|
| • | Polymerisationsinhibitor: | 0,01 | bis | 1,00 Gew.-% |
| • | Restliche Komponenten: | 0,10 | bis | 5,00 Gew.-%. |

Das Schleppmittel Cyclohexan wird in einer solchen Menge zudosiert, dass in dem Reaktor (1) der Reaktoreinheit (24) eine Konzentration an Cyclohexan im Bereich von 10 bis 90 Gew.-% entsteht, wobei sich diese Angabe des Gew.-%-Anteils auf die in dem Reaktor (1) der Reaktoreinheit (24) vorhandenen Komponenten inklusive dem Cyclohexan bezieht.

Zudem weisen in dieser bevorzugten Ausgestaltung die ohne das Schleppmittel Cyclohexan und ohne das Veresterungswasser aus dem Reaktor (1) der Reaktoreinheit (24) insgesamt herausströmenden Komponenten, nämlich den resultierenden, flüssigen Reaktionsaustrag plus den aus dem Reaktionsgemisch verdampften und aus der Reaktoreinheit (24) abgeführten Anteil, folgende Gewichtsanteile auf:

| | | | | |
|---|---|---|---|---|
| • | 2-Octyl(meth)acrylat: | 50,00 | bis | 95,00 Gew.-% |
| • | 2-Octanol: | 1,00 | bis | 30,00 Gew.-% |
| • | (Meth)acrylsäure: | 1,00 | bis | 15,00 Gew.-% |
| • | saurer Veresterungskatalysator: | 0,50 | bis | 10,00 Gew.-% |
| • | Polymerisationsinhibitor: | 0,01 | bis | 1,00 Gew.-% |
| • | Restliche Komponenten: | 2,49 | bis | 10,00 Gew.-%. |

Das sich durch das Cyclohexan sowie das Veresterungswasser bildende heterogene Azeotrop in einer Konzentration im Bereich von 10 bis 50 Gew.-% aus dem Reaktor (1) der Reaktoreinheit (24) herausströmt, wobei sich diese Angabe des Gew.-%-Anteils auf die insgesamt herausströmenden Komponenten inklusive des Cyclohexans und des Veresterungswassers bezieht.

In einer besonders bevorzugten Ausgestaltung weisen die ohne das Schleppmittel Cyclohexan in den einen Reaktor (1) der Reaktoreinheit (24) insgesamt zufließenden Komponenten folgende Gewichtsanteile auf:

| | | | | |
|---|---|---|---|---|
| • | 2-Octanol: | 50,00 | bis | 74,39 Gew.-% |
| • | (Meth)acrylsäure: | 25,00 | bis | 45,00 Gew.-% |
| • | saurer Veresterungskatalysator: | 0,50 | bis | 5,00 Gew.-% |
| • | Polymerisationsinhibitor: | 0,01 | bis | 1,00 Gew.-% |
| • | Restliche Komponenten: | 0,10 | bis | 2,00 Gew.-%. |

Das Schleppmittel Cyclohexan wird in einer solchen Menge zudosiert, dass in dem Reaktor (1) der Reaktoreinheit (24) eine Konzentration an Cyclohexan im Bereich von 10 bis 50 Gew.-% entsteht, wobei sich diese Angabe des Gew.-%-Anteils auf die in dem Reaktor (1) der Reaktoreinheit (24) vorhandenen Komponenten inklusive dem Cyclohexan bezieht.

Zudem weisen in dieser besonders bevorzugten Ausgestaltung die ohne das Schleppmittel Cyclohexan und ohne das Veresterungswasser aus den einen Reaktor (1) der Reaktoreinheit (24) insgesamt herausströmenden Komponenten, nämlich den resultierenden, flüssigen Reaktionsaustrag plus den aus dem Reaktionsgemisch verdampften und aus der Reaktoreinheit (24) abgeführten Anteil, folgende Gewichtsanteile auf:

| | | | | |
|---|---|---|---|---|
| • | 2-Octyl(meth)acrylat: | 70,00 | bis | 95,00 Gew.-% |
| • | 2-Octanol: | 1,00 | bis | 15,00 Gew.-% |
| • | (Meth)acrylsäure: | 1,00 | bis | 10,00 Gew.-% |
| • | saurer Veresterungskatalysator: | 0,50 | bis | 10,00 Gew.-% |
| • | Polymerisationsinhibitor: | 0,01 | bis | 1,00 Gew.-% |
| • | Restliche Komponenten: | 2,49 | bis | 10,00 Gew.-%. |

Das sich durch das Cyclohexan sowie das Veresterungswasser bildende heterogene Azeotrop strömt in einer Konzentration im Bereich von 10 bis 50 Gew.-% aus dem einen Reaktor (1) der Reaktoreinheit (24) heraus, wobei sich diese Angabe des Gew.-%-Anteils auf die insgesamt herausströmenden Komponenten inklusive des Cyclohexans und des Veresterungswassers bezieht.

Die Erfindung wird im Folgenden mit Verweis auf die Zeichnungen näher erläutert. Die Zeichnungen sind als Prinzipdarstellungen zu verstehen. Sie stellen keine Beschränkung der Erfindung dar, beispielsweise im Hinblick auf konkrete Abmessungen oder Ausgestaltungsvarianten.

Es zeigen:

Fig. 1:    Verfahrensskizze zur Herstellung von 2-Octyl(meth)acrylat basierend auf einem Verdampfer als Reaktorheizelement 30 zur Abtrennung des heterogenen Azeotrops.

Fig. 2:    Verfahrensskizze zur Herstellung von 2-Octyl(meth)acrylat basierend auf einem Verdampfer als Reaktorheizelement 30 und einer Azeotrop-Rektifikationskolonneneinheit 25 zur Abtrennung des heterogenen Azeotrops.

Fig. 3:    Verfahrensskizze zur Trennwandkolonne.


Liste der verwendeten Bezugszeichen:

| | |
|---|---|
| 1 | Reaktor 1 |
| 2 | Reaktor 2 |
| 3 | Reaktor 3 |
| 4 | Azeotrop-Rektifikationskolonne |
| 5 | Kondensator für das heterogene Azeotrop |
| 6 | Phasenscheider für das heterogene Azeotrop |
| 7 | Neutralisations-Extraktionseinheit |
| 8 | Wasserwäsche-Extraktionseinheit |
| 9 | Schleppmittel-Rektifikationskolonne |
| 10 | 2-Octanol-Rektifikationskolonne |
| 110 | 2-Octanol-Verdampfer |
| 11 | Reinsieder-Rektifikationskolonne |
| 111 | Reinsieder-Verdampfer |
| 12 | Schwersieder-Rektifikationskolonne |
| | |
| 112 | Schwersieder-Verdampfer |
| 13 | Zuführung von 2-Octanol |
| 14 | Zuführung von (Meth)acrylsäure |
| 15 | Zuführung von saurem Veresterungskatalysator |
| 16 | Abführung von Wasser, welches aus dem Phasenscheider (6) abgeführt wird. |
| 17 | Zuführung von Cyclohexan |
| 18 | Zuführung von Wasser für die Wasserwäsche |
| 19 | Zuführung von alkalischer Lösung |
| 20 | Abführung von Wasser mit Reste von Salzen aus der alkalischen und/oder der Wasserwäsche Extraktion |
| 21 | Abführung des Wertprodukts 2-Octyl(meth)acrylat |
| 22 | Abführung von Schwersieder, die bei der Schwersieder-Abtrennung im Sumpf entstehen. |
| 23 | Abführung von Leichtsieder, die bei der Cyclohexan-Abtrennung nach dem Austrag des Cyclohexan angereicherten Massenstroms aus dem Verfahren entfernt werden. |
| 24 | Reaktoreinheit |
| 25 | Azeotrop-Rektifikationskolonneneinheit |
| 30 | Reaktorheizelement |
| 31 | Zuführung von Polymerisationsinhibitor und/oder weiterer Stabilisatoren |
| | |
| 131 | Trennwandkolonne |
| 132 | Aufzutrennendes Gemisch der Trennwandkolonne |
| 134 | Destillatstrom der Trennwandkolonne |
| 135 | Teilstrom der Trennwandkolonne |
| 136 | Kondensator der Trennwandkolonne |
| 137 | Verdampfer der Trennwandkolonne |
| 138 | Trennwand der Trennwandkolonne |
| 139 | Gemeinsamer oberer Kolonnenbereich der Trennwandkolonne |
| 140 | Zulaufteil der Trennwandkolonne |

141      Entnahmeteil der Trennwandkolonne

142      Abtriebsteil der Trennwandkolonne

143      Verstärkungsteil der Trennwandkolonne

144      Unteren Kolonnenbereich der Trennwandkolonne

145      Brüdenstrom der Trennwandkolonne

146      Rücklaufstrom der Trennwandkolonne

147      Flüssigkeitsstrom der Trennwandkolonne

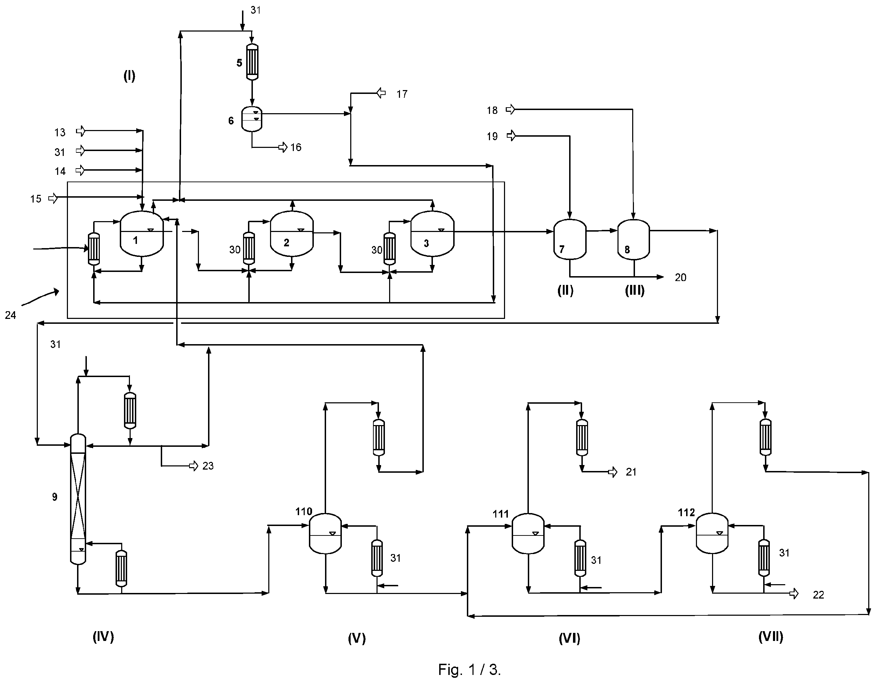

Fig. 1 zeigt eine Verfahrensskizze zur Herstellung von 2-Octyl(meth)acrylat mit den jeweiligen Verfahrensstufen, wobei jeder Reaktor ein Verdampfer als Reaktorheizelement zur Abtrennung des heterogenen Azeotrops enthält.

Verfahrensstufe (I): Veresterung

Die Veresterung wird in einer Reaktoreinheit 24 aus drei kaskadierten Reaktoren 1, 2, 3 umgesetzt. In den ersten Reaktor 1 der Kaskade strömen die Eduktströme: 2-Octanol 13, (Meth)acrylsäure 14, saurer Veresterungskatalysator 15, Polymerisationsinhibitoren 31 und das Schleppmittel Cyclohexan 17 ein. Nachdem der erste Reaktor 1 der Kaskade eine vorbestimmte Füllstandshöhe erreicht, fließt ein Massenstrom zum zweiten Reaktor 2 der Kaskade. Nachdem der zweite Reaktor 2 der Kaskade eine vorbestimmte Füllstandshöhe erreicht, fließt ein Massenstrom zum dritten Reaktor 3 der Kaskade. Nachdem der dritte Reaktor 3 eine vorbestimmte Füllstandshöhe erreicht, fließt ein resultierender Reaktionsaustrag aus der Reaktionseinheit heraus. Alle drei Reaktoren 1, 2, 3 der Reaktoreinheit 24 enthalten jeweils einen eigenen Verdampfer 30, durch den das Reaktionsgemisch durchströmt, erhitzt und anschließend dem jeweiligen Reaktor zurückgeführt wird. Im Verdampfer 30 verdampft teilweise das sich bei der Veresterung bildende Veresterungswasser und das Schleppmittel Cyclohexan als heterogenes Azeotrop (Cyclohexan / Veresterungswasser).

Ein mit dem heterogenen Azeotrop angereicherter Gasstrom wird den einzelnen Reaktoren 1, 2, 3 entnommen und durch einen nachgeschalteten Kondensator 5 bis zur Kondensation gekühlt. In einem nachgeschalteten Phasenscheider 6 bildet sich eine organische, obere Phase und eine wässrige, untere Phase.

Die wässrige untere Phase wird aus dem Verfahren ausgeschleust. Die organische, obere Phase enthält zu 50 bis 99,9 Gew.-% Cyclohexan. Diese organische, obere Phase wird dem ersten Reaktor 1 der Kaskade und der Azeotrop-Rektifikationskolonne 4 im Bereich vom Kopf bis zur Mitte der Kolonne zugeführt.

Verfahrensstufe (II): Alkalische Extraktion

Der resultierende Reaktionsaustrag aus der Veresterungsstufe wird mit einer alkalischen Lösung 19 innerhalb einer Neutralisations-Extraktionseinheit 7 neutralisiert, wobei der saure Veresterungskatalysator sowie nicht umgesetzte (Meth)acrylsäure neutralisiert werden. Dadurch entsteht eine obere Neutralisations-Phase, welche 2-Octyl(meth)acrylat enthält, und eine untere Neutralisations-Phase, welche durch die Neutralisation erzeugte Salze sowie Wasser enthält. Die untere Neutralisations-Phase 20 wird aus dem Verfahren ausgeschleust.

Verfahrensstufe (III): Wasserwäsche-Extraktion

Im Anschluss an die alkalische Extraktion wird die obere Neutralisations-Phase einer Wasserwäsche-Extraktionseinheit 8 zugeführt, wobei unter Zufuhr von Wasser 18 eine mit Wasser und Reste von Salzen enthaltende untere Wasserwäsche-Phase 20 und eine mit 2-Octyl(meth)acrylat enthaltende obere Wasserwäsche-Phase gebildet werden. Die untere Wasserwäsche-Phase 20 wird aus dem Verfahren ausgeschleust.

Verfahrensstufe (IV): Cyclohexan-Abtrennung

Die obere Wasserwäsche-Phase von der Wasserwäsche-Extraktion wird einer Schleppmittel-Rektifikationskolonne 9 zugeführt. Über den Kopf dieser Kolonne 9 werden Leichtsieder und Cyclohexan abgezogen.

Die über den Kopf der Kolonne 9 abgezogenen Komponenten werden im Bereich von 95 bis 99,9 Gew.-% kondensiert und der Schleppmittel-Rektifikationskolonne 9 im Kopfbereich zurückgeführt. Der sich ergebende restliche Teil wird nach Abtrennung von gasförmigen Leichtsiedern 23, wie Octene, dem ersten Reaktor 1 der Kaskade zugeführt. Typischerweise wird Cyclohexan aus der Kolonne 9 zum ersten Reaktor 1 der Kaskade im Bereich von 2 bis 20 Gew.-%, bezogen auf die dem Verfahren insgesamt zugeführte Cyclohexan Gesamtmasse, gefördert.

Verfahrensstufe (V): 2-Octanol-Abtrennung

Der nicht in die Schleppmittel-Rektifikationskolonne 9 zurückgeführte Teil des Sumpfaustrags wird zu einem 2-Octanol-Verdampfer 110 geführt. Über den 2-Octanol-Verdampfer 110 wird 2-Octanol in einer Menge im Bereich von 20 bis 60 Gew.-%, bezogen auf den dem 2-Octanol-Verdampfer 110 zugeführten Sumpfaustrag der Schleppmittel-Rektifikationskolonne 9, abgezogen, kondensiert und vollständig dem ersten Reaktor 1 der Kaskade zugeführt.

Verfahrensstufe (VI): Reinsieder-Abtrennung

Der nicht in den 2-Octanol-Verdampfer 110 zurückgeführte Teil des Sumpfaustrags wird zu einem Reinsieder-Verdampfer 111 geführt. Über einen Brüdenstrom des Reinsieder-Verdampfers 111 wird das Wertprodukt 2-Octyl(meth)acrylat im Bereich von 80 bis 95 Gew.-%, bezogen auf den dem Reinsieder-Verdampfer 111 zugeführten Sumpfaustrag, abgezogen, kondensiert und vollständig dem Reinsieder-Verdampfer 111 zurückgeführt. Der sich ergebende restliche Teil des Sumpfaustrags des Octanol-Verdampfers 110 wird kondensiert und vollständig einem Schwersieder-Verdampfer 112 zugeführt.

Verfahrensstufe (VII): Schwersieder-Abtrennung

Der entsprechend restliche Teil des Sumpfaustrags des Reinsieder-Verdampfers 111 wird zu einem Schwersieder-Verdampfer 112 geführt. Über einen Brüdenstrom des Schwersieder-Verdampfers 112 werden Leichtsieder im Bereich von 40 bis 90 Gew.-%, bezogen auf den dem Schwersieder-Verdampfer 112 zugeführten Sumpfaustrag des Reinsieder-Verdampfers 111, abgezogen und vollständig dem Reinsieder-Verdampfer 111 zurückgeführt. Die im Sumpf des Schwersieder-Verdampfers 112 befindlichen Schwersieder 22 werden zu 1 bis 10 Gew.-%, bezogen auf den Umlauf des Schwersieder-Verdampfers 112, aus dem Verfahren herausgeschleust, wobei der sich ergebende restliche Teil der Schwersieder 22 verdampft wird und zum Schwersieder-Verdampfer 112 zurückgeführt wird.

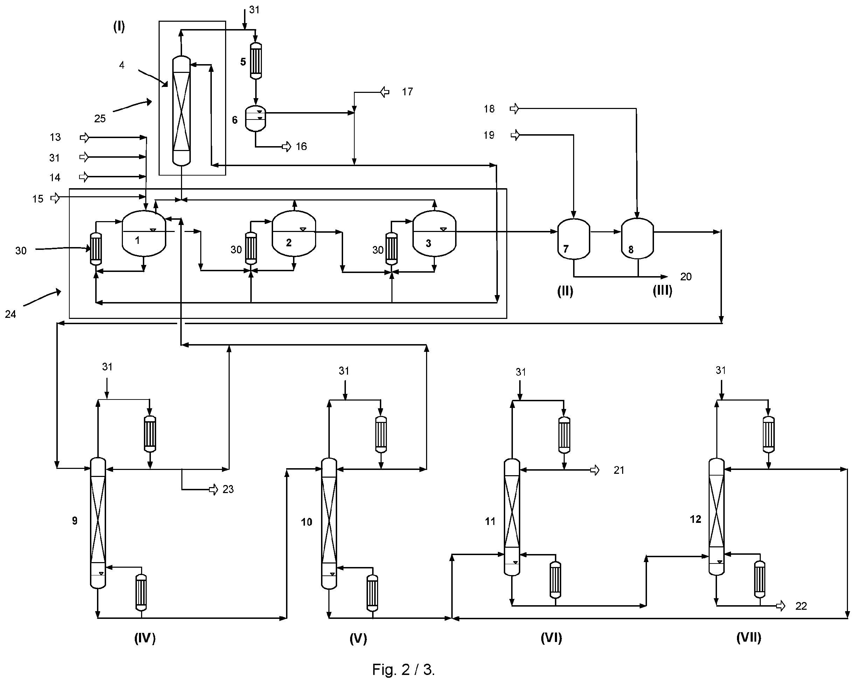

Fig. 2 zeigt eine Verfahrensskizze zur Herstellung von 2-Octyl(meth)acrylat mit den jeweiligen Verfahrensstufen, wobei eine Azeotrop-Rektifikationskolonne 4 zur Abtrennung des heterogenen Azeotrops verwendet wird.

Verfahrensstufe (I): Veresterung

Die Veresterung wird in einer Reaktoreinheit 24 aus drei kaskadierten Reaktoren 1, 2, 3 umgesetzt. In den ersten Reaktor 1 der Kaskade strömen die Eduktströme: 2-Octanol 13, (Meth)acrylsäure 14, saurer Veresterungskatalysator 15, Polymerisationsinhibitoren 31 und das Schleppmittel Cyclohexan 17 ein. Nachdem der erste Reaktor 1 der Kaskade eine vorbestimmte Füllstandshöhe erreicht, fließt ein Massenstrom zum zweiten Reaktor 2 der Kaskade. Nachdem der zweite Reaktor 2 der Kaskade eine vorbestimmte Füllstandshöhe erreicht, fließt ein Massenstrom zum dritten Reaktor 3 der Kaskade. Nachdem der dritte Reaktor 3 eine vorbestimmte Füllstandshöhe erreicht, fließt ein resultierender Reaktionsaustrag aus der Reaktionseinheit heraus. Alle drei Reaktoren 1, 2, 3 der Reaktoreinheit 24 enthalten jeweils einen eigenen Verdampfer 30, durch den das Reaktionsgemisch durchströmt, erhitzt und anschließend dem jeweiligen Reaktor zurückgeführt wird. Im Verdampfer 30 verdampft zumindest teilweise das sich bei der Veresterung bildende Veresterungswasser und das Schleppmittel Cyclohexan als heterogenes Azeotrop (Cyclohexan / Veresterungswasser).

Ein Gasstrom, welcher mit dem heterogenen Azeotrop angereichert ist, wird den einzelnen Reaktoren 1, 2, 3 entnommen und einer nachgeschalteten Azeotrop-Rektifikationskolonne 4 zugeführt. Die Azeotrop-Rektifikationskolonne 4 ist hierbei auf die Reaktoreinheit 24 aufgesetzt. Am Kopf der Azeotrop-Rektifikationskolonne 4 werden Polymerisationsinhibitoren 31 zugeführt. Der mit heterogenem Azeotrop angereicherte Gasstrom wird über den Kopf der Azeotrop-Rektifikationskolonne 4 geführt und durch einen Kondensator 5 bis zur Kondensation gekühlt. In einem nachgeschalteten Phasenscheider 6 bildet sich eine organische, obere Phase und eine wässrige, untere Phase.

Die wässrige untere Phase wird aus dem Verfahren ausgeschleust. Die organische, obere Phase enthält zu 50 bis 99,9 Gew.-% Cyclohexan. Diese organische, obere Phase wird dem ersten Reaktor 1 der Kaskade und der Azeotrop-Rektifikationskolonne 4 im Bereich vom Kopf bis zur Mitte der Kolonne zugeführt.

Verfahrensstufe (II): Alkalische Extraktion

Der resultierende Reaktionsaustrag aus der Veresterungsstufe wird mit einer alkalischen Lösung innerhalb einer Neutralisations-Extraktionseinheit 7 neutralisiert, wobei der saure Veresterungskatalysator sowie nicht umgesetzte (Meth)acrylsäure neutralisiert werden. Dadurch entsteht eine obere Neutralisations-Phase, welche 2-Octyl(meth)acrylat enthält, und eine untere Neutralisations-Phase, welche durch die Neutralisation erzeugte Salze sowie Wasser enthält. Die untere Neutralisations-Phase wird aus dem Verfahren ausgeschleust.

Verfahrensstufe (III): Wasserwäsche-Extraktion

Im Anschluss an die alkalische Extraktion wird die obere Neutralisations-Phase einer Wasserwäsche-Extraktionseinheit 8 zugeführt, wobei unter Zufuhr von Wasser 18 eine mit Wasser und Reste von Salzen enthaltende untere Wasserwäsche-Phase 20 und eine mit 2-Octyl(meth)acrylat enthaltende obere Wasserwäsche-Phase gebildet werden. Die untere Wasserwäsche-Phase 20 wird aus dem Verfahren ausgeschleust.

Verfahrensstufe (IV): Cyclohexan-Abtrennung

Die obere Wasserwäsche-Phase von der Wasserwäsche-Extraktion wird einer Schleppmittel-Rektifikationskolonne 9 zugeführt. Über den Kopf dieser Kolonne 9 werden Leichtsieder und Cyclohexan abgezogen.
Die über den Kopf der Kolonne 9 abgezogenen Komponenten werden im Bereich von 95 bis 99,9 Gew.-% kondensiert und der Schleppmittel-Rektifikationskolonne 9 im Kopfbereich zurückgeführt. Der sich ergebende restliche Teil wird nach Abtrennung von Leichtsiedern 23, wie Octene, dem ersten Reaktor 1 der Kaskade zugeführt. Typischerweise wird Cyclohexan aus der Kolonne 9 zum ersten Reaktor 1 der Kaskade im Bereich von 2 bis 20 Gew.-%, bezogen auf die dem Verfahren insgesamt zugeführte Cyclohexan Gesamtmasse, gefördert.

Verfahrensstufe (V): 2-Octanol-Abtrennung

Der nicht in die Schleppmittel-Rektifikationskolonne 9 zurückgeführte Teil des Sumpfaustrags wird zu einer 2-Octanol-Rektifikationskolonne 10 geführt. Über den Kopf dieser Kolonne 10 wird 2-Octanol in einer Menge im Bereich von 20 bis 60 Gew.-%, bezogen auf den der Kolonne 10 zugeführten Sumpfaustrag der Schleppmittel-Rektifikationskolonne 9, abgezogen. Die über den Kopf der Kolonne 10 abgezogenen Komponenten werden vollständig kondensiert und im Bereich von 0 bis 50 Gew.-% der 2-Octanol-Rektifikationskolonne 10 im Kopfbereich zurückgeführt, wohingegen der sich ergebende restliche Teil des Kopfabzugs dem ersten Reaktor 1 der Kaskade zugeführt wird.

Verfahrensstufe (VI): Reinsieder-Abtrennung

Der nicht in die 2-Octanol-Rektifikationskolonne 10 zurückgeführte Teil des Sumpfaustrags wird zu einer Reinsieder-Rektifikationskolonne 11 geführt. Über den Kopf dieser Reinsieder-Rektifikationskolonne 11 wird das Wertprodukt 2-Octyl(meth)acrylat im Bereich von 80 bis 95 Gew.-%, bezogen auf den der Kolonne 11 zugeführten Sumpfaustrag, abgezogen. Der Austrag über den Kopf der Reinsieder-Rektifikationskolonne 11 wird vollständig kondensiert und im Bereich von 0 bis 60 Gew.-% in den Kopfbereich der Reinsieder-Rektifikationskolonne 11 zurückgeführt. Der sich ergebende restliche Teil des Sumpfaustrags der Octanol-Rektifikationskolonne 10 wird einer Schwersieder-Abtrennung zugeführt.

Verfahrensstufe (VII): Schwersieder-Abtrennung

Der entsprechend restliche Teil des Sumpfaustrags der Reinsieder-Rektifikationskolonne 11 wird zu einer Schwersieder-Rektifikationskolonne 12 geführt. Über den Kopf dieser Schwersieder-Rektifikationskolonne 12 werden Leichtsieder im Bereich von 40 bis 300 Gew.-%, bezogen auf den der Schwersieder-Rektifikationskolonne 12 zugeführten Sumpfaustrag, abgezogen und ein Teil des Abzugs im Bereich von 40 bis 90 Gew.-% wird der Reinsieder-Rektifikationskolonne 11 zurückgeführt, wobei der sich ergebende restliche Teil der Schwersieder-Rektifikationskolonne 12 im Kopfbereich der Kolonne 12 zurückgeführt wird. Die im Sumpf der Schwersieder-Rektifikationskolonne 12 befindlichen Schwersieder 22 werden zumindest teilweise aus dem Verfahren herausgeschleust, wobei der sich ergebende restliche Teil der Schwersieder 22 verdampft wird und im Sumpfbereich der Schwersieder-Rektifikationskolonne 12 zurückgeführt wird.

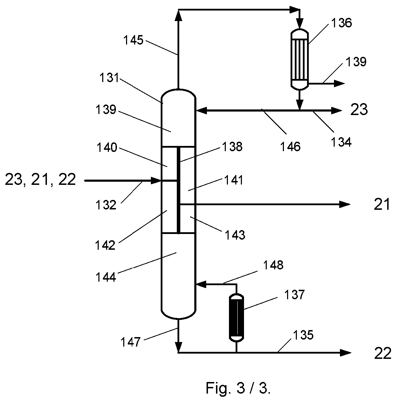

Fig. 3 zeigt eine Verfahrensskizze zur Trennwandkolonne.
Die Verfahrensskizze zeigt eine Trennwandkolonne 131 mit Trennwand 138, die die Trennwandkolonne 131 in einen gemeinsamen oberen Kolonnenbereich 139, einen Zulaufteil 140, 142, mit Verstärkungsteil 140 und Abtriebsteil 142 einen Entnahmeteil 141, 143 mit einem Abtriebsteil 141 und einem Verstärkungsteil 143 sowie einen gemeinsamen unteren Kolonnenbereich 144 unterteilt. Das aufzutrennende Gemisch 132, das Leichtsieder, 2-Octyl(meth)acrylat und Schwersieder enthält, tritt zwischen den Kolonnenteilen 140 und 142 in die Trennwandkolonne 131 ein. Das Reinprodukt 133 wird zwischen den Kolonnenteilen 141 und 143, bevorzugt in flüssiger Form, entnommen. Der am Kolonnenkopf anfallende Brüdenstrom 145 wird im Kondensator 136, der gegebenenfalls durch einen Nachkühler ergänzt wird, teilweise kondensiert und in den Rücklaufstrom 146 sowie den Destillatstrom 134 aufgeteilt. Der nicht kondensierte Anteil aus dem Kondensator 136 enthält die leichtsiedenden Verunreinigungen und wird dampfförmig als Strom 139 abgezogen. Am unteren Kolonnenende wird die Flüssigkeit 147 in einem Verdampfer 137 teilweise verdampft und über die Rohrleitung 148 in die Kolonne zurückgeführt. Ein Teilstrom 135, der die schwersiedenden Verunreinigungen enthält, wird abgezogen. Der Verdampfer 137 kann als Naturumlaufverdampfer oder als Zwangsumlaufverdampfer ausgebildet sein, im letzteren Fall ist zusätzlich eine Umlaufpumpe für den Flüssigkeitsstrom 147 erforderlich. Besonders vorteilhaft hinsichtlich der Vermeidung unerwünschter Polymerisationsreaktionen ist es anstelle des Zwangsumlaufverdampfers einen Fallfilmverdampfer oder Dünnschichtverdampfer einzusetzen, da mit dieser Bauart die kürzesten Verweilzeiten möglich sind.

Zur Verringerung der Verweilzeit der Flüssigkeit im Verdampfersystem ist es günstig, die Standhaltung nicht in der unteren Kolonnenhaube, sondern in der Zulaufleitung des Flüssigkeitsstroms 147 anzuordnen.

Beispiele

Sowohl in den folgenden Beispielen als auch in dieser gesamten Schrift bezieht sich der Begriff "ein qualitatives GC" auf eine Gaschromatografie (GC) bei der die einzelnen Komponenten durch Retentionszeitvergleich zugeordnet wurden. Die Konzentration der jeweiligen Komponente im Gemisch wird dabei als prozentualer Peakflächenanteil (Fl.-%) angenommen.

Sowohl in den folgenden Beispielen als auch in dieser gesamten Schrift bezieht sich der Begriff "ein quantitatives GC" auf eine Gaschromatografie (GC) mit Quantifizierung unter Bezug auf den internen Standard n-Tetradecan. Es wurden die Komponenten 2-Octylacrylat, Cyclohexan und 2-Octanol quantifiziert. Die Konzentration der jeweiligen Komponente im Gemisch wird als Gewichtsprozent (Gew.-%) erhalten.

Die erwartete Wassermenge entspricht der Wassermenge bei Vollumsatz bzw. unendlicher Reaktionszeit und ergibt sich aus der Summe der Wassergehalte der eingesetzten Startmaterialien und dem zu bildenden Veresterungswasser bei 100 % Umsatz. Die Summe aus diesen Wassergehalten wird in dieser Schrift auch als Reaktionswasser bezeichnet. Das maximal zu bildende Veresterungswasser kann über die gebildeten Mole Wasser berechnet werden, die äquivalent zu den eingesetzten Molen Acrylsäure sind. Nicht in der Bilanz berücksichtigt wurde ein geringfügiges Verschleppen der Acrylsäure in die wässrige Phase.

Beispiel 1: Diskontinuierliche Veresterung mit Cyclohexan (Verfahrensstufe I)

In einem mit Thermofühler, Ankerrührer, Wasserauskreiser, Intensivkühler und Lufteinleitung ausgestatteten, beheizbaren 0,75 L-Doppelwandreaktor wurden 300 g 2-Octanol, 0,6 g Phenothiazin, 0,6 g einer 1 Gew.-%-igen Lösung von HO-TEMPO in 2-Octylacrylat, 130 g Cyclohexan, 0,015 g CuCl und 0,240 g 50 %-ige unterphosphorige Säure vorgelegt. Anschließend wurden 161 g Acrylsäure zudosiert, wobei die Acrylsäure mit 200 ppm MeHQ stabilisiert war. 6,4 g 100 %-ige Methansulfonsäure wurden hinzugefügt. Die Gesamtansatzgröße betrug 599 g.

Die Reaktionsmischung wurde mit einer Badtemperatur von 125 °C aufgeheizt. Mit dem Aufheizen startete die Reaktionszeit. Im Verlauf der Reaktion wurden Sumpfproben gezogen und gaschromatografisch analysiert, um den Verlauf der Reaktion zu beobachten.

Nach 0,58 h begann bei einer Sumpftemperatur von 99 °C Wasser überzugehen und die Destillationszeit startete. Die Reaktion wurde nach 6h Destillationszeit, gleichbedeutend mit 6,59 h Reaktionszeit, beim Erreichen einer Sumpftemperatur von 119 °C abgebrochen. Es wurden 35,6 g Wasser abdestilliert, entsprechend 88,2 % der erwarteten Wassermenge.

Das Reaktionsgemisch wurde mittels Gaschromatografie nach 6 h Destillationszeit analysiert. 2-Octanol sowie 2-Octylacrylat wurden quantifiziert. Die Mischung enthielt nach 6 h Destillationszeit, gleichbedeutend mit 6,59 h Reaktionszeit, 71,54 Gew.-% Octylacrylat und 5,75 Gew.-% 2-Octanol. Das Reaktionsgemisch enthielt 0,27 Fl.-% Octene, 1,78 Fl.-% Diacrylsäureester sowie 1,85 Fl.-% Oxiester.

Der Umsatz betrug 92,56% bezogen auf 2-Octanol.

Vergleichsbeispiel 1: Diskontinuierliche Veresterung mit Toluol (Verfahrensstufe I)

In einem mit Thermofühler, Ankerrührer, Wasserauskreiser, Intensivkühler und Lufteinleitung ausgestatteten, beheizbaren 0,75 L-Doppelwandreaktor wurden 300 g 2-Octanol, 0,6 g Phenothiazin, 0,6 g einer 1 Gew.-%-igen Lösung von HO-TEMPO in 2-Octylacrylat, 130 g Toluol, 0,015 g CuCl und 0,240 g 50 %-ige unterphosphorige Säure vorgelegt. Anschließend wurden 161 g Acrylsäure zudosiert, wobei die Acrylsäure mit 200 ppm MeHQ stabilisiert war. 6,4 g 100 %-ige Methansulfonsäure wurden hinzugefügt. Die Gesamtansatzgröße betrug 599 g.

Die Reaktionsmischung wurde mit einer Badtemperatur von 125 °C aufgeheizt. Mit dem Aufheizen startete die Reaktionszeit. Im Verlauf der Reaktion wurden Sumpfproben gezogen und gaschromatografisch analysiert, um den Verlauf der Reaktion zu beobachten.

Nach 0,92 h begann bei einer Sumpftemperatur von 108 °C Wasser überzugehen und die Destillationszeit startete. Die Reaktion wurde nach 6h Destillationszeit, gleichbedeutend mit 6,92 h Reaktionszeit, beim Erreichen einer Sumpftemperatur von 116 °C abgebrochen. Es wurden 17,7 g Wasser abdestilliert, entsprechend 43,9 % der erwarteten Wassermenge.

Das Reaktionsgemisch wurde mittels Gaschromatografie nach 6 h Destillationszeit analysiert. 2-Octanol sowie 2-Octylacrylat wurden quantifiziert. Die Mischung enthielt nach 6 h Destillationszeit, gleichbedeutend mit 6,92 h Reaktionszeit, 58,38 Gew.-% Octylacrylat und 13,35 Gew.-% 2-Octanol. Das Reaktionsgemisch enthielt 0,15 Fl.-% Octene, 2,18 Fl.-% Diacrylsäureester sowie 1,57 Fl.-% Oxiester.

Der Umsatz betrug 81,39 % bezogen auf 2-Octanol.

Vergleichsbeispiel 2: Diskontinuierliche Veresterung mit 2-Octanol als Schleppmittel (Verfahrensstufe I)

In einem mit Thermofühler, Ankerrührer, Wasserauskreiser, Intensivkühler und Lufteinleitung ausgestatteten, beheizbaren 0,75 L-Doppelwandreaktor wurden 300 g 2-Octanol, 0,6 g Phenothiazin, 0,6 g einer 1 Gew.-%-igen Lösung von HO-TEMPO in 2-Octylacrylat, weitere 130 g 2-Octanol, 0,015 g CuCl und 0,240 g 50 %-ige unterphosphorige Säure vorgelegt. Anschließend wurden 161 g Acrylsäure zudosiert, wobei die Acrylsäure mit 200 ppm MeHQ stabilisiert war. 6,4 g 100 %-ige Methansulfonsäure wurden hinzugefügt. Die Gesamtansatzgröße betrug 599 g.

Die Reaktionsmischung wurde mit einer Badtemperatur von 125 °C aufgeheizt. Mit dem Aufheizen startete die Reaktionszeit. Im Verlauf der Reaktion wurden Sumpfproben gezogen und gaschromatografisch analysiert, um den Verlauf der Reaktion zu beobachten.

Nach 1,22 h begann bei einer Sumpftemperatur von 112 °C Wasser überzugehen und die Destillationszeit startete. Die Reaktion wurde nach 6 h Destillationszeit, gleichbedeutend mit 7,22 h Reaktionszeit beim Erreichen einer Sumpftemperatur von 114 °C abgebrochen. Es wurden 12,8 g Wasser abdestilliert, entsprechend 31,6 % der erwarteten Wassermenge.

Das Reaktionsgemisch wurde mittels Gaschromatografie nach 6 h Destillationszeit analysiert. 2-Octanol sowie 2-Octylacrylat wurden quantifiziert. Die Mischung enthielt nach 6 h Destillationszeit, gleichbedeutend mit 7,22 h Reaktionszeit, 54,64 Gew.-% Octylacrylat und 33,01 Gew.-% 2-Octanol. Das Reaktionsgemisch enthielt 0,09 Fl.-% Octene, 1,11 Fl.-% Diacrylsäureester sowie 2,20 Fl.-% Oxiester.

Der Umsatz betrug 62,35 % bezogen auf 2-Octanol.

Vergleichsbeispiel 3: Diskontinuierliche Veresterung ohne Schleppmittel (Verfahrensstufe I)

In einem mit Thermofühler, Ankerrührer, Wasserauskreiser, Intensivkühler und Lufteinleitung ausgestatteten, beheizbaren 0,75 L-Doppelwandreaktor wurden 383 g 2-Octanol, 0,6 g Phenothiazin, 0,6 g einer 1 %-igen Lösung von Hydroxy-Tempo in 2-Octylacrylat, 0,015 g CuCl und 0,240 g 50-%ige unterphosphorige Säure vorgelegt. Anschließend wurden 206 g Acrylsäure zudosiert, wobei die Acrylsäure mit 200 ppm MeHQ stabilisiert war. 8,2 g 100 %-ige Methansulfonsäure wurden hinzugefügt. Die Gesamtansatzgröße betrug 599 g.

Die Reaktionsmischung wurde mit einer Badtemperatur von 125 °C aufgeheizt. Mit dem Aufheizen startete die Reaktionszeit. Im Verlauf der Reaktion wurden Sumpfproben gezogen und gaschromatografisch analysiert, um den Verlauf der Reaktion zu beobachten.

Nach 1,17 h begann bei einer Sumpftemperatur von 111 °C Wasser überzugehen und die Destillationszeit startete. Die Reaktion wurde nach 6 h Destillationszeit, gleichbedeutend mit 7,17 h Reaktionszeit, beim Erreichen einer Sumpftemperatur von 113 °C abgebrochen. Es wurden 11,0 g Wasser abdestilliert, entsprechend 21,4 % der erwarteten Wassermenge.

Das Reaktionsgemisch wurde mittels Gaschromatografie nach 6 h Destillationszeit analysiert. 2-Octanol sowie 2-Octylacrylat wurden quantifiziert. Die Mischung enthielt nach 6 h Destillationszeit, gleichbedeutend mit 7,17 h Reaktionszeit, 59,16 Gew.-% Octylacrylat und 21,33 Gew.-% 2-Octanol. Das Reaktionsgemisch enthielt 0,06 Fl.-% Octene, 2,57 Fl.-% Diacrylsäureester sowie 2,00 Fl.-% Oxiester.

Der Umsatz betrug 73,5 % bezogen auf 2-Octanol.

Zusammenfassung

Alle Versuche wurden bei einer Badtemperatur von 125 °C durchgeführt. Säure und Alkohol wurden im gleichen Molverhältnis eingesetzt. Eine Ausnahme stellt hier der Versuch mit Schleppmittel 2-Octanol dar, bei dem das Molverhältnis Acrylsäure zu 2-Octanol 1: 1,45 betrug. Die Katalysatorkonzentration bezogen auf die eingesetzte Säure war immer gleich.

Schleppmittel waren in den Versuchen Cyclohexan, Toluol und 2-Octanol, das zusätzlich zum 2-Octanol für die Reaktion vorhanden war. Zudem wurde in einem Versuch kein Schleppmittel verwendet.

Tabellarische Zusammenstellung der Ergebnisse

Die Tabelle 1 zeigt eine Übersicht zur Reaktion beim Beispiel 1, welches das Schleppmittel Cyclohexan verwendet. Aufgeführt werden die Destillationszeit, die Reaktionszeit, die Sumpftemperatur, das Reaktionswasser und der Umsatz. Dieser wasserbasierte Umsatz wurde wie folgt berechnet:

$$\text{wasserbasierter } Umsatz \text{ [\%]} = \frac{Reaktionswasser\ [g]\ (t=x\,h)}{Reaktionswasser\ [g]\ (t=\infty)} \times 100.$$

Hierbei bedeutet Reaktionswasser mit t = ∞ die maximal zu erwartende Menge an Reaktionswasser, die bei vollständigem Umsatz der Veresterung vorliegt. Das Reaktionswasser mit t = x h entspricht der Menge an Reaktionswasser, das zum Zeitpunkt x Stunden aufgrund der Veresterung und des Wassergehalts der eingesetzten Startmaterialien vorliegt.

Tabelle 1 : Übersicht zur Reaktion beim Beispiel 1, welches das Schleppmittel Cyclohexan verwendet. Aufgeführt werden die Destillationszeit, die Reaktionszeit, die Sumpftemperatur, das Reaktionswasser und der wasserbasierte Umsatz.

| Destillationszeit [h] | 0 | 1 | 2 | 3 | 4 | 5 | 6 |
|---|---|---|---|---|---|---|---|
| Reaktionszeit [h] | 0,58 | 1,58 | 2,58 | 3,58 | 4,58 | 5,58 | 6,58 |
| Sumpftemperatur [°C] | 99,2 | 107,4 | 112,8 | 116,1 | 117,7 | 118,5 | 118,9 |
| Reaktionswasser [g] | | 16,57 | 28,63 | 33,08 | 34,44 | 35,12 | 35,6 |
| wasserbasierter Umsatz [%] | | 41,07 | 70,95 | 81,98 | 85,35 | 87,04 | 88,23 |

Die Tabelle 2 zeigt eine Übersicht der GC-Werte der qualitativen GC-Messungen zu verschiedenen Destillationszeiten zum Beispiel 1, welches das Schleppmittel Cyclohexan verwendet. Aufgeführt werden die Summe aus 1-Octen und 2-Octen, der Diacrylsäureester (DIAS-Ester), der Oxiester, der Gehalt an 2-Octylacrylat, der Umsatz, berechnet aus den Werten des qualitativen GC, und das Verhältnis 2-Octylacrylat zur Summe der Nebenprodukte 1-Octen, 2-Octen, DIAS-Ester und Oxiester.

Der Umsatz wurde hierbei wie folgt berechnet:

$$Umsatz \text{ [\%]} = \frac{GC-Wert\ (2-Octylacrylat)\ [Fl.-\%]}{GC-Wert\ (2-Octylacrylat)[Fl.-\%] + GC-Wert\ (2-Octanol)\ [Fl.-\%]} \times 100.$$

Hierbei bezeichnet der GC-Wert (2-Octylacrylat) [Fl.-%] den mittels qualitativem GC gemessenen Flächenprozentanteil an 2-Octylacrylat. Analog bezeichnet der GC-Wert (2-Octanol) [Fl.-%] den mittels qualitativem GC gemessenen Flächenprozentanteil an 2-Octanol.

Tabelle 2: Übersicht der GC-Werte der qualitativen GC-Messungen zu verschiedenen Destillationszeiten zum Beispiel 1, welches das Schleppmittel Cyclohexan verwendet. Aufgeführt werden die Summe aus 1-Octen und 2-Octen, der DIAS-Ester, der Oxiester, der Gehalt an 2-Octylacrylat, der Umsatz berechnet aus den Werten des qualitativen GC und das Verhältnis 2-Octylacrylat zur Summe der Nebenprodukte 1-Octen, 2-Octen, DIAS-Ester und Oxiester.

| Destillationszeit [h] | 1 | 2 | 3 | 4 | 5 | 6 |
|---|---|---|---|---|---|---|
| Summe 1-Octen, 2-Octen [Fl.-%] | 0 | 0 | 0,1 | 0,15 | 0,21 | 0,27 |
| DIAS-Ester [Fl.-%] | 1,05 | 1,34 | 1,55 | 1,7 | 1,76 | 1,78 |
| Oxiester [Fl.-%] | 0,64 | 1,06 | 1,39 | 1,65 | 1,76 | 1,85 |
| Gehalt 2-Octylacrylat [Fl.-%] | 40,14 | 55,61 | 64,07 | 70,88 | 70,06 | 69,89 |
| Umsatz [%] | 57,07 | 77,35 | 86,36 | 90,13 | 91,59 | 92,36 |
| Verhältnis 2-Octylacrylat zur Summe der Nebenprodukte 1-Octen, 2-Octen, DIAS-Ester, Oxiester | 100 : 4,2 | 100 : 4,3 | 100 : 4,7 | 100 : 4,9 | 100 : 5,3 | 100 : 5,6 |

Die Tabelle 3 zeigt eine Übersicht zu verschiedenen Destillationszeiten zum Beispiel 1, welches das Schleppmittel Cyclohexan verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.

Der molare Umsatz wurde wie folgt berechnet:

$$Molarer\ Umsatz\ [\%] =$$

$$\frac{GC\text{-}Wert\ (2\text{-}Octylacrylat)\ [Gew.\text{-}\%]/G\_Mol\ (2\text{-}Octylacrylat)[\frac{g}{mol}]}{GC\text{-}Wert\ (2\text{-}Octylacrylat)[Gew.\text{-}\%]/\ G\_Mol\ (2\text{-}Octylacrylat)[\frac{g}{mol}]+GC\text{-}Wert\ (2\text{-}Octanol)\ [Gew.\text{-}\%]/\ G\_Mol\ (2\text{-}Octanol)[\frac{g}{mol}]} \times 100,$$

mit der Abkürzung G_Mol als Molgewicht.

Hierbei bezeichnet der GC-Wert (2-Octylacrylat) [Gew.-%] den mittels Gaschromatografie quantifizierten Anteil an 2-Octylacrylat. Analog bezeichnet der GC-Wert (2-Octanol) [Gew.-%] den mittels Gaschromatografie quantifizierten Anteil an 2-Octanol.

Tabelle 3: Übersicht der GC-Werte zu verschiedenen Destillationszeiten zum Beispiel 1, welches das Schleppmittel Cyclohexan verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.

| Destillationszeit [h] | 3 | 4 | 5 | 6 |
|---|---|---|---|---|
| 2-Octylacrylat [Gew.-%] | 66,61 | 70,27 | 70,70 | 71,54 |
| 2-Octanol [Gew.-%] | 10,20 | 7,66 | 6,38 | 5,75 |
| Molarer Umsatz [%] | 82,18 | 86,63 | 88,67 | 89,79 |

Die Tabelle 4 zeigt eine Übersicht zur Reaktion beim Vergleichsbeispiel 1, welches das Schleppmittel Toluol verwendet. Aufgeführt werden die Destillationszeit, die Reaktionszeit, die Sumpftemperatur, das Reaktionswasser und der Umsatz. Dieser wasserbasierte Umsatz wurde wie folgt berechnet:

$$\textbf{wasserbasierter}\ Umsatz\ [\%] = \frac{Reaktionsswasser\ [g]\ (t=x\ h)}{Reaktionswasser\ [g]\ (t=\infty)} \times 100.$$

Hierbei bedeutet Reaktionswasser mit $t = \infty$ die maximal zu erwartende Menge an Reaktionswasser, die bei vollständigem Umsatz der Veresterung vorliegt. Das Reaktionswasser mit t = x h entspricht der Menge an Reaktionswasser, das zum Zeitpunkt x Stunden aufgrund der Veresterung und des Wassergehalts der eingesetzten Startmaterialien vorliegt.

Tabelle 4 : Übersicht zur Reaktion beim Vergleichsbeispiel 1, welches das Schleppmittel Toluol verwendet. Aufgeführt werden die Destillationszeit, die Reaktionszeit, die Sumpftemperatur, das Reaktionswasser und der wasserbasierte Umsatz.

| Destillationszeit [h] | 0 | 1 | 2 | 3,00 | 4 | 5 | 6 |
|---|---|---|---|---|---|---|---|
| Reaktionszeit [h] | 0,92 | 1,92 | 2,92 | 3,92 | 4,92 | 5,92 | 6,92 |
| Sumpftemperatur [°C] | 107,8 | 111,4 | 113,1 | 114,1 | 115,1 | 115,6 | 115,9 |
| Reaktionswasser [g] | | 5,97 | 10,95 | 13,87 | 16,2 | 17,09 | 17,73 |
| wasserbasierter Umsatz [%] | | 14,80 | 27,14 | 34,37 | 40,15 | 42,35 | 43,94 |

Die Tabelle 5 zeigt eine Übersicht der GC-Werte der qualitativen GC-Messungen zu verschiedenen Destillationszeiten zum Vergleichsbeispiel 1, welches das Schleppmittel Toluol verwendet. Aufgeführt werden die Summe aus 1-Octen und 2-Octen, der DIAS-Ester, der Oxiester, der Gehalt an 2-Octylacrylat, der Umsatz, berechnet aus den Werten des qualitativen GC, und das Verhältnis 2-Octylacrylat zur Summe der Nebenprodukte 1-Octen, 2-Octen, DIAS-Ester und Oxiester.

Der Umsatz wurde hierbei wie folgt berechnet:

$$Umsatz\ [\%] = \frac{GC\text{-}Wert\ (2\text{-}Octylacrylat)\ [Fl.\text{-}\%]}{GC\text{-}Wert\ (2\text{-}Octylacrylat)[Fl.\text{-}\%]+GC\text{-}Wert\ (2\text{-}Octanol)\ [Fl.\text{-}\%]} \times 100.$$

Hierbei bezeichnet der GC-Wert (2-Octylacrylat) [Fl.-%] den mittels qualitativem GC gemessenen Flächenprozentanteil an 2-Octylacrylat. Analog bezeichnet der GC-Wert (2-Octanol) [Fl.-%] den mittels qualitativem GC gemessenen Flächenprozentanteil an 2-Octanol.

Tabelle 5: Übersicht der GC-Werte der qualitativen GC-Messungen zu verschiedenen Destillationszeiten zum Vergleichsbeispiel 1, welches das Schleppmittel Toluol verwendet. Aufgeführt werden die Summe aus 1-Octen und 2-Octen, der DIAS-Ester, der Oxiester, der Gehalt an 2-Octylacrylat, der Umsatz berechnet aus den Werten des qualitativen GC und das Verhältnis 2-Octylacrylat zur Summe der Nebenprodukte 1-Octen, 2-Octen, DIAS-Ester und Oxiester.

| Destillationszeit [h] | 1 | 2 | 3 | 4,00 | 5 | 6 |
|---|---|---|---|---|---|---|
| Summe 1-Octen, 2-Octen [Fl.-%] | | | 0,08 | 0,11 | 0,12 | 0,15 |
| DIAS-Ester [Fl.-%] | | | 1,59 | 1,85 | 2,00 | 2,18 |
| Oxiester [Fl.-%] | | | 1,24 | 1,38 | 1,47 | 1,57 |
| Gehalt 2-Octylacrylat [Fl.-%] | | | 51,68 | 54,43 | 55,84 | 56,78 |
| Umsatz [%] | | | 73,96 | 77,56 | 79,28 | 80,79 |
| Verhältnis 2-Octylacrylat zur Summe der Nebenprodukte 1-Octen, 2-Octen, DIAS-Ester, Oxiester | | | 100 : 5,6 | 100 : 6,1 | 100 : 6,4 | 100 : 6,9 |

Die Tabelle 6 zeigt eine Übersicht zu verschiedenen Destillationszeiten zum Vergleichsbeispiel 1, welches das Schleppmittel Toluol verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.

Der molare Umsatz wurde wie folgt berechnet:

$$Molarer\ Umsatz\ [\%] = \frac{GC-Wert\ (2-Octylacrylat)\ [Gew.-\%]/G\_Mol\ (2-Octylacrylat)[\frac{g}{mol}]}{GC-Wert\ (2-Octylacrylat)[Gew.-\%]/\ G\_Mol\ (2-Octylacrylat)[\frac{g}{mol}] + GC-Wert\ (2-Octanol)\ [Gew.-\%]/\ G\_Mol\ (2-Octanol)[\frac{g}{mol}]} \times 100,$$

mit der Abkürzung G_Mol als Molgewicht.

Hierbei bezeichnet der GC-Wert (2-Octylacrylat) [Gew.-%] den mittels Gaschromatografie quantifizierten Anteil an 2-Octylacrylat. Analog bezeichnet der GC-Wert (2-Octanol) [Gew.-%] den mittels Gaschromatografie quantifizierten Anteil an 2-Octanol.

Tabelle 6: Übersicht der GC-Werte zu verschiedenen Destillationszeiten zum Vergleichsbeispiel 1, welches das Schleppmittel Toluol verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.

| Destillationszeit [h] | 3 | 4 | 5 | 6 |
|---|---|---|---|---|
| 2-Octylacrylat [Gew.-%] | 52,87 | 55,90 | 57,62 | 58,38 |
| 2-Octanol [Gew.-%] | 17,87 | 15,53 | 14,46 | 13,35 |
| Molarer Umsatz [%] | 67,64 | 71,77 | 73,79 | 75,55 |

Die Tabelle 7 zeigt eine Übersicht zur Reaktion beim Vergleichsbeispiel 2, welches das Schleppmittel 2-Octanol verwendet. Aufgeführt werden die Destillationszeit, die Reaktionszeit, die Sumpftemperatur, das Reaktionswasser und der Umsatz. Dieser wasserbasierte Umsatz wurde wie folgt berechnet:

$$wasserbasierter\ Umsatz\ [\%] = \frac{Reaktionswasser\ [g]\ (t=x\ h)}{Reaktionswasser\ [g]\ (t=\infty)} \times 100.$$

Hierbei bedeutet Reaktionswasser mit $t = \infty$ die maximal zu erwartende Menge an Reaktionswasser, die bei vollständigem Umsatz der Veresterung vorliegt. Das Reaktionswasser mit t = x h entspricht der Menge an Reaktionswasser, das zum Zeitpunkt x Stunden aufgrund der Veresterung und des Wassergehalts der eingesetzten Startmaterialien vorliegt.

Tabelle 7 : Übersicht zur Reaktion beim Vergleichsbeispiel 2, welches das Schleppmittel 2-Octanol verwendet. Aufgeführt werden die Destillationszeit, die Reaktionszeit, die Sumpftemperatur, das Reaktionswasser und der wasserbasierte Umsatz.

| Destillationszeit [h] | 0 | 1 | 2 | 3 | 4 | 5 | 6 |
|---|---|---|---|---|---|---|---|
| Reaktionszeit [h] | 1,22 | 2,22 | 3,22 | 4,22 | 5,22 | 6,22 | 7,22 |
| Sumpftemperatur [°C] | 112,1 | 111,1 | 111,6 | 112,1 | 113,2 | 113,7 | 114,3 |
| Reaktionswasser [g] | | 1,24 | 3,34 | 6,09 | 8,96 | 11,02 | 12,76 |
| wasserbasierter Umsatz [%] | | 3,07 | 8,28 | 15,09 | 22,21 | 27,31 | 31,62 |

Die Tabelle 8 zeigt eine Übersicht der GC-Werte der qualitativen GC-Messungen zu verschiedenen Destillationszeiten zum Vergleichsbeispiel 2, welches das Schleppmittel 2-Octanol verwendet. Aufgeführt werden die Summe aus 1-Octen und 2-Octen, der DIAS-Ester, der Oxiester, der Gehalt an 2-Octylacrylat, der Umsatz, berechnet aus den Werten des qualitativen GC, und das Verhältnis 2-Octylacrylat zur Summe der Nebenprodukte 1-Octen, 2-Octen, DIAS-Ester und Oxiester.

Der Umsatz wurde hierbei wie folgt berechnet:

$$Umsatz\ [\%] = \frac{GC-Wert\ (2-Octylacrylat)\ [Fl.-\%]}{GC-Wert\ (2-Octylacrylat)[Fl.-\%] + GC-Wert\ (2-Octanol)\ [Fl.-\%]} \times 100.$$

Hierbei bezeichnet der GC-Wert (2-Octylacrylat) [Fl.-%] den mittels qualitativem GC gemessenen Flächenprozentanteil an 2-Octylacrylat. Analog bezeichnet der GC-Wert (2-Octanol) [Fl.-%] den mittels qualitativem GC gemessenen Flächenprozentanteil an 2-Octanol.

Tabelle 8: Übersicht der GC-Werte der qualitativen GC-Messungen zu verschiedenen Destillationszeiten zum Vergleichsbeispiel 2, welches das Schleppmittel 2-Octanol verwendet. Aufgeführt werden die Summe aus 1-Octen und 2-Octen, der DIAS-Ester, der Oxiester, der Gehalt an 2-Octylacrylat, der Umsatz berechnet aus den Werten des qualitativen GC und das Verhältnis 2-Octylacrylat zur Summe der Nebenprodukte 1-Octen, 2-Octen, DIAS-Ester und Oxiester.

| Destillationszeit [h] | 1 | 2 | 3 | 4 | 5 | 6 |
|---|---|---|---|---|---|---|
| Summe 1-Octen, 2-Octen [Fl.-%] | | | 0,12 | 0,08 | 0,04 | 0,09 |
| DIAS-Ester [Fl.-%] | | | 0,92 | 0,99 | 1,04 | 1,11 |
| Oxiester [Fl.-%] | | | 1,62 | 1,82 | 2,00 | 2,20 |
| Gehalt 2-Octylacrylat [Fl.-%] | | | 51,46 | 53,71 | 55,20 | 56,46 |
| Umsatz [%] | | | 55,40 | 57,82 | 59,57 | 60,99 |
| Verhältnis 2-Octylacrylat zur Summe der Nebenprodukte 1-Octen, 2-Octen, DIAS-Ester, Oxiester | | | 100 : 5,2 | 100 : 5,4 | 100 : 5,6 | 100 : 6,0 |

Die Tabelle 9 zeigt eine Übersicht zu verschiedenen Destillationszeiten zum Vergleichsbeispiel 2, welches das Schleppmittel 2-Octanol verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.
Der molare Umsatz wurde wie folgt berechnet:

$$Molarer\ Umsatz\ [\%] =$$
$$\frac{GC-Wert\ (2-Octylacrylat)\ [Gew.-\%]/G\_Mol\ (2-Octylacrylat)[\frac{g}{mol}]}{GC-Wert\ (2-Octylacrylat)[Gew.-\%]/\ G\_Mol\ (2-Octylacrylat)[\frac{g}{mol}] + GC-Wert\ (2-Octanol)\ [Gew.-\%]/\ G\_Mol\ (2-Octanol)[\frac{g}{mol}]} \times 100,$$

mit der Abkürzung G_Mol als Molgewicht.
Hierbei bezeichnet der GC-Wert (2-Octylacrylat) [Gew.-%] den mittels Gaschromatografie quantifizierten Anteil an 2-Octylacrylat. Analog bezeichnet der GC-Wert (2-Octanol) [Gew.-%] den mittels Gaschromatografie quantifizierten Anteil an 2-Octanol.

Tabelle 9: Übersicht der GC-Werte zu verschiedenen Destillationszeiten zum Vergleichsbeispiel 2, welches das Schleppmittel 2-Octanol verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.

| Destillationszeit [h] | 3 | 4 | 5 | 6 |
|---|---|---|---|---|
| 2-Octylacrylat [Gew.-%] | 49,31 | 51,63 | 53,43 | 54,66 |
| 2-Octanol [Gew.-%] | 37,50 | 35,49 | 34,17 | 33,01 |
| Molarer Umsatz [%] | 56,81 | 50,69 | 52,48 | 53,91 |

Die Tabelle 10 zeigt eine Übersicht zur Reaktion beim Vergleichsbeispiel 3, welches kein Schleppmittel verwendet. Aufgeführt werden die Destillationszeit, die Reaktionszeit, die Sumpftemperatur, das Reaktionswasser und der Umsatz. Dieser wasserbasierte Umsatz wurde wie folgt berechnet:

$$\text{wasserbasierter } Umsatz\ [\%] = \frac{Reaktionswasser\ [g]\ (t=x\ h)}{Reaktionswasser\ [g]\ (t=\infty)} \times 100.$$

Hierbei bedeutet Reaktionswasser mit $t = \infty$ die maximal zu erwartende Menge an Reaktionswasser, die bei vollständigem Umsatz der Veresterung vorliegt. Das Reaktionswasser mit $t = x\ h$ entspricht der Menge an Reaktionswasser, das zum Zeitpunkt x Stunden aufgrund der Veresterung und des Wassergehalts der eingesetzten Startmaterialien vorliegt.

Tabelle 10 : Übersicht zur Reaktion beim Vergleichsbeispiel 3, welches kein Schleppmittel verwendet. Aufgeführt werden die Destillationszeit, die Reaktionszeit, die Sumpftemperatur, das Reaktionswasser und der wasserbasierte Umsatz.

| Destillationszeit [h] | 0 | 1 | 2 | 3 | 4 | 5 | 6 |
|---|---|---|---|---|---|---|---|
| Reaktionszeit [h] | 1,17 | 2,17 | 3,17 | 4,17 | 5,17 | 6,17 | 7,17 |
| Sumpftemperatur [°C] | 110,7 | 110,2 | 110,6 | 111,1 | 111,7 | 112,0 | 112,5 |
| Reaktionswasser [g] | | 1,33 | 3,23 | 5,67 | 8,21 | 9,51 | 11,00 |
| wasserbasierter Umsatz [%] | | 2,58 | 6,27 | 11,01 | 15,95 | 18,47 | 21,37 |

Die Tabelle 11 zeigt eine Übersicht der GC-Werte der qualitativen GC-Messungen zu verschiedenen Destillationszeiten zum Vergleichsbeispiel 3, welches kein Schleppmittel verwendet. Aufgeführt werden die Summe aus 1-Octen und 2-Octen, der DIAS-Ester, der Oxiester, der Gehalt an 2-Octylacrylat, der Umsatz, berechnet aus den Werten des qualitativen GC, und das Verhältnis 2-Octylacrylat zur Summe der Nebenprodukte 1-Octen, 2-Octen, DIAS-Ester und Oxiester.

Der Umsatz wurde hierbei wie folgt berechnet:

$$Umsatz\ [\%] = \frac{GC-Wert\ (2-Octylacrylat)\ [Fl.-\%]}{GC-Wert\ (2-Octylacrylat)[Fl.-\%] + GC-Wert\ (2-Octanol)\ [Fl.-\%]} \times 100.$$

Hierbei bezeichnet der GC-Wert (2-Octylacrylat) [Fl.-%] den mittels qualitativem GC gemessenen Flächenprozentanteil an 2-Octylacrylat. Analog bezeichnet der GC-Wert (2-Octanol) [Fl.-%] den mittels qualitativem GC gemessenen Flächenprozentanteil an 2-Octanol.

Tabelle 11: Übersicht der GC-Werte der qualitativen GC-Messungen zu verschiedenen Destillationszeiten zum Vergleichseispiel 11, welches kein Schleppmittel verwendet. Aufgeführt werden die Summe aus 1-Octen und 2-Octen, der DIAS-Ester, der Oxiester, der Gehalt an 2-Octylacrylat, der Umsatz berechnet aus den Werten des qualitativen GC und das Verhältnis 2-Octylacrylat zur Summe der Nebenprodukte 1-Octen, 2-Octen, DIAS-Ester und Oxiester.

| Destillationszeit [h] | 1 | 2 | 3 | 4 | 5 | 6 |
|---|---|---|---|---|---|---|
| Summe 1-Octen, 2-Octen [Fl.-%] | | | 0,06 | 0,04 | 0,07 | 0,06 |
| DIAS-Ester [Fl.-%] | | | 1,82 | 2,13 | 2,34 | 2,57 |

(fortgesetzt)

| Destillationszeit [h] | 1 | 2 | 3 | 4 | 5 | 6 |
|---|---|---|---|---|---|---|
| Oxiester [Fl.-%] | | | 1,60 | 1,78 | 1,88 | 2,00 |
| Gehalt 2-Octylacrylat [Fl.-%] | | | 59,31 | 61,47 | 62,91 | 63,58 |
| Umsatz [%] | | | 67,13 | 69,69 | 71,2 | 72,41 |
| Verhältnis 2-Octylacrylat zur Summe der Nebenprodukte 1-Octen, 2-Octen, DIAS-Ester, Oxiester | | | 100 : 5,9 | 100 : 6,4 | 100 : 6,8 | 100 : 7,3 |

Die Tabelle 12 zeigt eine Übersicht zu verschiedenen Destillationszeiten zum Vergleichsbeispiel 3, welches kein Schleppmittel verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.

Der molare Umsatz wurde wie folgt berechnet:

$$Molarer\ Umsatz\ [\%] = \frac{GC\text{-}Wert\ (2\text{-}Octylacrylat)\ [Gew.\text{-}\%]/G\_Mol\ (2\text{-}Octylacrylat)[\frac{g}{mol}]}{GC\text{-}Wert\ (2\text{-}Octylacrylat)[Gew.\text{-}\%]/G\_Mol\ (2\text{-}Octylacrylat)[\frac{g}{mol}] + GC\text{-}Wert\ (2\text{-}Octanol)\ [Gew.\text{-}\%]/G\_Mol\ (2\text{-}Octanol)[\frac{g}{mol}]} \times 100,$$

mit der Abkürzung G_Mol als Molgewicht.

Hierbei bezeichnet der GC-Wert (2-Octylacrylat) [Gew.-%] den mittels Gaschromatografie quantifizierten Anteil an 2-Octylacrylat. Analog bezeichnet der GC-Wert (2-Octanol) [Gew.-%] den mittels Gaschromatografie quantifizierten Anteil an 2-Octanol.

Tabelle 12: Übersicht der GC-Werte zu verschiedenen Destillationszeiten zum Vergleichsbeispiel 3, welches kein Schleppmittel verwendet. Aufgeführt werden der Gewichtsanteil an 2-Octylacrylat, der Gewichtsanteil an 2-Octanol und der molare Umsatz. Hierbei wird der molare Umsatz aus den Werten des quantitativen GC berechnet.

| Destillationszeit [h] | 3 | 4 | 5 | 6 |
|---|---|---|---|---|
| 2-Octylacrylat [Gew.-%] | 54,47 | 56,46 | 57,96 | 59,16 |
| 2-Octanol [Gew.-%] | 25,28 | 23,19 | 22,32 | 21,33 |
| Molarer Umsatz [%] | 60,35 | 63,24 | 64,73 | 66,21 |

Die Konzentration der Nebenprodukte steigt im Verlauf der fortschreitenden Reaktion. Bei ähnlichem Umsatz ist die Bildung der Nebenprodukte bei Verwendung von Cyclohexan als Schleppmittel im Vergleich zu den eingesetzten Schleppmittel Toluol und 2-Octanol oder im Fall, dass kein Schleppmittel verwendet wird, deutlich geringer.

Die folgende Tabelle 13 vergleicht bei ähnlichen Umsätzen, die jeweils aus den GC-Werten des qualitativen GC bestimmt wurden, das Verhältnis von 2-Octylacrylat zur Summe der Nebenprodukte 1-Octen, 2-Octen, DIAS-Ester und Oxiester. Hierbei werden die Beispiele mit dem jeweiligen Schleppmittel Cyclohexan, Toluol und 2-Octanol sowie das Beispiel ohne Schleppmittel dargestellt.

Tabelle 13: Vergleich des Verhältnis von 2-Octylacrylat zur Summe der Nebenprodukte 1-Octen, 2-Octen, DIAS-Ester und Oxiester bei ähnlichen Umsätzen. Hierbei werden die Beispiele mit dem jeweiligen Schleppmittel Cyclohexan, Toluol und 2-Octanol sowie das Beispiel ohne Schleppmittel dargestellt.

| Cyclohexan | Umsatz [%] | 57,07 | 77,35 | 86,36 |
|---|---|---|---|---|
| | Verhältnis von 2-Octylacrylat zur Summe der Nebenprodukte 1-Octen, 2-Octen, DIAS-Ester, Oxiester | 100 : 4,2 | 100 : 4,3 | 100 : 4,7 |
| Toluol | Umsatz [%] | | 77,56 | 80,79 |
| | Verhältnis von 2-Octylacrylat zur Summe der Nebenprodukte 1-Octen, 2-Octen, DIAS-Ester, Oxiester | | 100 : 6,1 | 100 : 6,9 |

(fortgesetzt)

| 2-Octanol | Umsatz [%] | 55,40 | |
|---|---|---|---|
| | Verhältnis von 2-Octylacrylat zur Summe der Nebenprodukte 1-Octen, 2-Octen, DIAS-Ester, Oxiester | 100 : 5,2 | |
| Ohne Schleppmittel | Umsatz [%] | | 72,41 |
| | Verhältnis von 2-Octylacrylat zur Summe der Nebenprodukte 1-Octen, 2-Octen, DIAS-Ester, Oxiester | | 100 : 7,3 |

Daraus ergeben sich folgende Vorteile bei der Verwendung von Cyclohexan, wenn man die Ergebnisse mit den Vergleichsbeispielen 1, 2 und 3 vergleicht:

- Mit dem Schleppmittel Cyclohexan wird bei gleichem Energieeintrag ein höchster Umsatz generiert. Dadurch entstehen weniger Verluste an (Meth)Acrylsäure in der anschließenden alkalischen Extraktionseinheit (Verfahrensstufe II). Zudem wird weniger 2-Octanol rezykliert, kleinere Reaktoren bei gleichem Durchsatz
- Mit dem Schleppmittel Cyclohexan wird ein schnellster Start des Rückfluss nach Beginn des Aufheizens erreicht. Dadurch wird eine kürzere Reaktionszeit und weniger Energie benötigt.
- Das Schleppmittel Cyclohexan trennt am effizientesten das Wasser ab.
- Mit dem Schleppmittel Cyclohexan ist bei gleichem Reaktionsvolumen und gleicher Destillationszeit der Gehalt an 2-Octylacrylat im Reaktionsgemisch am größten. Damit ist die absolute Raum-Zeit Ausbeute am höchsten.
- Mit dem Schleppmittel Cyclohexan werden weniger Nebenprodukte bei gleichem Umsatz gebildet.

Vergleichsbeispiel 4: Veresterung, alkalische Extraktion und Wasserwäsche ohne Schleppmittel (Verfahrensstufe I, II und III)

In einem beheizbaren 0,75 L-Doppelwandreaktor, ausgestattet mit u.a. einem Thermofühler, einem Ankerrührer, einem Glasaufsatz der Firma Normag (Quelle: www.normag-glas.de), welcher einen Intensiv-Kühler, einen Ablaufstutzen mit zwei Nadelventile, einen Vakuumrahmen mit zwei Spindelventilen als Absperrventil und ein Belüftungsventil enthält, sowie eine Vakuumpumpe und eine Lufteinleitung wurden 300 g 2-Octanol, 0,52 g Phenothiazin, 0,012 g CuCl und 0,207 g 50 %-ige unterphosphorige Säure vorgelegt. Anschließend wurden 209 g Acrylsäure zudosiert, wobei die Acrylsäure mit 200 ppm MeHQ stabilisiert war. 6,4 g 100 %-ige Methansulfonsäure wurden hinzugefügt. Es wurde mit einer Badtemperatur von 130 °C aufgeheizt, wodurch die Reaktionszeit startete. Nach einer Stunde begann bei einer Sumpftemperatur von 112 °C Wasser überzugehen und die Destillationszeit startete. Nach 2 h Destillationszeit wurde begonnen, den Absolutdruck in 50 mbar-Schritten bis auf 100 mbar zu reduzieren. Die Sumpfinnentemperatur betrug maximal 122 °C. Nach 4,1 h Destillationszeit wurde die Reaktion abgebrochen. Es wurden 71,8 g abdestilliert. Die Reaktionsmischung wurde nach dem Abkühlen mit Wasser, mit 12,5 %-iger NaOH-Lösung und anschließend wieder mit Wasser extrahiert. Nach Phasentrennung wurde die organische Phase mit 50 mg MeHQ versetzt. Es wurden 403 g 2-Octylacrylat mit einer Reinheit von 85,4 GC-Fl.-% erhalten. Es waren noch 3,5 Fl.-% 2-Octanol enthalten sowie 5,9 Fl.-% Diacrylsäureester und 3,1 Fl.-% Oxiester. Das zweiphasige Destillat wurde zur Einphasenbildung mit Ethanol versetzt und mittels qualitativem GC sowie Säure-Base-Titration und Karl-Fischer-Titration analysiert. Die Zusammensetzung des Destillats vor der Ethanolzugabe wurde basierend auf diesen Ergebnissen rechnerisch bestimmt. Das Destillat setzte sich zusammen aus 51,8 % Wasser, 29,5 % Acrylsäure sowie 6,3 % 2-Octanol und 9,5 % 2-Octylacrylat, außerdem 2,2 % Octenen.

Beispiel 2: Veresterung, alkalische Extraktion und Wasserwäsche mit Cyclohexan (Verfahrensstufe I, II und III)

In einem beheizbaren 4 L-Doppelwandreaktor, ausgestattet mit u.a. einem Thermofühler, einem Scheibenrührer, einem Wasserauskreiser, einem Intensivkühler, einer Lufteinleitung, wurden 1503 g 2-Octanol, 4,01 g Phenothiazin und 1,94 g MeHQ vorgelegt. Anschließend wurden 1082 g Acrylsäure zudosiert, wobei die Acrylsäure mit 200 ppm MeHQ stabilisiert war. 65,7 g p-Toluolsulfonsäuremonohydrat sowie 1189 g Cyclohexan wurden hinzugefügt und es wurde aufgeheizt. Bei einer Sumpftemperatur im Bereich von 92 bis 104 °C ging Wasser über. Nach 7,2 h wurde die Reaktion abgebrochen. Die Reaktionsmischung wurde nach dem Abkühlen mit Wasser, mit 12,5 %i-ger NaOH-Lösung und anschließend wieder mit Wasser extrahiert. Nach Phasentrennung wurde die organische Phase mit 250 mg MeHQ versetzt und im Vakuum konzentriert. Es wurden 2072 g 2-Octylacrylat mit einer Reinheit von 93,2 GC-Fl.-% erhalten. Es waren noch 2,6 Fl.-% 2-Octanol enthalten sowie 2,0 Fl.-% Diacrylsäureester und 1,3 Fl.-% Oxiester. Eine GC-Analytik der im Wasserauskreiser vorhandenen organischen Flüssigkeit zeigte 99,38 GC-Fl.-% Cyclohexan sowie 0,47 Fl.-% Acrylsäure, 0,03 Fl.-% 2-Octanol, 0,1 Fl.-% 2-Octylacrylat und 0,01 Fl.-% Octene. Eine Karl-Fischer Titration des wässrigen Destillats ergab 85 Gew.-% Wasser.

Beispiel 3: Veresterung, alkalische Extraktion und Wasserwäsche mit Cyclohexan (Verfahrensstufe I, II und III)

In diesem Beispiel 3 wurde u.a. ein Reaktor 1 und eine Azeotrop-Rektifikationskolonne 4 verwendet. Die Verfahrensstufe I, II und III zur Veresterung, zur alkalischen Extraktion und zur Wasserwäsche-Extraktion wurden kontinuierlich betrieben.

Ein beheizbarer 1,6 L Doppelwandglasreaktor 1 war u.a. mit einem beheizbaren Deckel sowie mit einem 3-stufigen Kreuzbalkenrührer ausgestattet.

Als Azeotrop-Rektifikationskolonne 4 wurde eine doppelwandige, verspiegelte Glaskolonne mit den Maßen 50 cm (Höhe) x 43 mm (Durchmesser) mit einer strukturierten Packung von Montz (Quelle: www.montz.de/gewebepackung-typ-3a , aufgerufen am 1.11.2021) verwendet (Montz Pak Typ A3-750, 3x 150 mm x 41 mm).

Der Aufbau umfasste weiterhin einen Kühler, einen Phasenscheider 6 , eine Magerlufteinleitung, Behälter und Vorrichtungen für Edukt-Dosierung, Behälter und Vorrichtungen für das Heteroazeotrop, eine Vorlage für den Sumpfaustrag sowie eine Vorlage für die Stabilisierung der Kolonne.

Zunächst wurde durch einen diskontinuierlichen Versuch ein stabilisiertes Reaktionsgemisch hergestellt, welches im Wesentlichen aus 2-Octanol, Acrylsäure sowie 2-Octylacrylat und Katalysator Methansulfonsäure bestand. Die Startmaterialien zur Herstellung dieses Reaktionsgemischs wurden in den Verhältnissen eingesetzt, die dem später zudosierten Startgemisch entsprachen. Das Startgemisch zur Herstellung des Reaktionsgemischs enthielt 45 Gew.-% 2-Octanol, 25 Gew.-% Acrylsäure rein, 0,93 Gew.-% 100%-ige Methansulfonsäure, 29 Gew.-% Cyclohexan, 0,04 Gew.-% als 100 %-ig berechnete hypophosphorige Säure, 0,1 Gew.-% Phenothiazin. sowie 0,002 Gew.-% CuCl. Die Herstellung erfolgte analog zu Beispiel 2.

Die Badtemperatur wurde auf 128 °C eingestellt, die des Deckels auf 95 °C.

Es wurde in Summe 667 g/h Startgemisch in den ersten Reaktor 1 dosiert. Das Startgemisch kann dabei in einem oder mehreren Behälter vorgelegt werden, wobei sich die in dem Behälter befindlichen Substanzgemische in ihrer Zusammensetzung unterscheiden können. In Summe bestand das Startgemisch aus 45 Gew.-% 2-Octanol, 25 Gew.-% Acrylsäure rein, 0,93 Gew.-% 100%ige Methansulfonsäure, 29 Gew.-% Cyclohexan, 0,04 Gew.-% hypophosphorige Säure (ber. 100%) sowie 0,1 Gew.-% Phenothiazin. Zusätzlich wurde in regelmäßigen Abständen CuCl als Feststoff dosiert, so dass die durchschnittliche Konzentration 0,002 Gew.-% betrug.

Außerdem wurden 350 g/h Cyclohexan, stabilisiert mit HO-TEMPO, auf den Kopf der Kolonne dosiert. Füllstands-kontrolliert wurde kontinuierlich Material dem Reaktor 1 entnommen. Der Inhalt des Reaktors (1) wurde regelmäßig mittels Gaschromatografie analysiert.

Als sich ein Gleichgewicht und eine konstante Sumpftemperatur von 108 °C einstellte, wurde der Behälter für den Reaktionsaustrag gewechselt, das Material des Vorlaufs verworfen und der neue Reaktionsaustrag gesammelt.

Dieses Material wurde in einem zweiten Versuch in derselben Geschwindigkeit, wie die Entnahme im ersten Versuch erfolgte, dem noch mit Reaktionsgemisch des vorhergehenden Versuchs gefüllten Reaktor 1 zugeführt. Außerdem wurden 150 g/h Cyclohexan in den Sumpf sowie 50 g/h Cyclohexan (stabilisiert mit HO-TEM PO) auf den Kopf der Kolonne dosiert. Wieder wurde füllstands-kontrolliert kontinuierlich Material dem Reaktor 1 entnommen.

Sobald sich ein Gleichgewicht und eine konstante Sumpftemperatur von 113 °C eingestellt hatte, wurde der Behälter für den Reaktionsaustrag gewechselt, das Material des Vorlaufs verworfen und der neue Reaktionsaustrag gesammelt.

Dieses Material wurde in einem dritten Versuch in derselben Geschwindigkeit, wie die Entnahme im zweiten Versuch erfolgte, dem noch mit Reaktionsgemisch des vorhergehenden Versuchs gefüllten Reaktor 1 zugeführt. Außerdem wurden 150 g/h Cyclohexan in den Sumpf sowie 50 g/h Cyclohexan (stabilisiert mit HO-TEMPO) auf den Kopf der Kolonne dosiert. Wieder wurde füllstands-kontrolliert kontinuierlich Material dem Reaktor 1 entnommen.

Sobald sich ein Gleichgewicht und eine konstante Sumpftemperatur von 114 °C eingestellt hatte, wurde der Behälter für den Reaktionsaustrag gewechselt, das Material des Vorlaufs verworfen und der neue Reaktionsaustrag gesammelt.

Dieses Material wurde mittels Säure-Base-Titration sowie qualitativer und quantitativer Gaschromatografie untersucht. Hierbei wurden Octylacrylat, Cyclohexan und 2-Octanol über eine Gaschromatografie quantifiziert, wohingegen Acrylsäure und Methansulfonsäure über eine Säure-Base Titration ermittelt wurden. Der Gehalt an den Nebenkomponenten Octene, Diacrylsäureester sowie Oxiester wurden über ein qualitatives GC bestimmt. Bei den Nebenkomponenten, die nur zu einem geringen Anteil im Reaktionsgemisch vorliegen, wurde des Weiteren angenommen, dass die als Flächenprozent ermittelten Werte den Werten entsprechen, die man durch eine Quantifizierung erhalten würde.

Dieser Reaktionsaustrag enthielt 67,6 Gew.-% 2-Octylacrylat, 22,4 Gew.-% Cyclohexan, Gew.-3,1 Gew.-% 2-Octanol, 2,4 Gew.-% Acrylsäure, 1,1 Gew.-% Methansulfonsäure, 0,26 Gew.-% Octene, 1,1 Gew.-% Diacrylsäureester sowie 1,5 Gew.-% Oxiester.

Der Reaktionsaustrag wurde einer kontinuierlichen Neutralisation innerhalb einer alkalischen Extraktionseinheit 7 und anschließend einer Wasserwäsche innerhalb einer Wasserwäsche-Extraktionseinheit 8 unterworfen.

Die alkalischen Extraktionseinheit 7 enthält eine kontinuierliche Apparatur für die Neutralisation mit 10%iger Natronlauge NaOH. Diese kontinuierliche Apparatur besteht aus einer Mischpumpe und einem Phasenscheider 6 mit einem Durchmesser von 40 mm und einer Trennschichtregelung.

Bei einer Temperatur von 30°C wurden 0,76 kg/h der verdünnten Natronlauge NaOH und 3,3 kg/h des Reaktionsaustrags über die Mischpumpe dispergiert und im Phasenscheider 6 getrennt. Die neutralisierte organische Phase wurde dann in der Wasserwäsche-Extraktionseinheit 8 mit 5 kg/h Wasser durch einem statischen Mischer (Kenics-Mischer (https://de.wikipedia.org/wiki/Statischer_Mischer , aufgerufen am 23.11.2021) mit 4,9 mm Innendurchmesser und 27 Elementen) dispergiert und anschließend in einem Phasenscheider 6 mit einem Durchmesser von DN40 getrennt.

Beispiel 4: Destillation (Verfahrensstufen IV bis VII)

Die destillative Reinigung von 2-Octylacrylat wurde in vier nachfolgenden, kontinuierlich betriebenen Verfahrensstufen durchgeführt:

- Cyclohexan-Abtrennung (IV)
- 2-Octanol- Abtrennung (V)
- Reinsieder-Abtrennung (VI)
- Schwersieder- Abtrennung (VII)

Der Versuchsaufbau war für jede einzelne Verfahrensstufe IV bis VII gleich und enthielt jeweils einen Dünnschichtverdampfer mit einer Fläche von 0,016 Quadratmeter, welcher in der jeweiligen Rektifikationskolonne 9, 10, 11, 12 eingesetzt wurde. Die jeweilige Rektifikationskolonne 9, 10, 11, 12 hatte einen Durchmesser von 30 mm und eine Packungslänge von 100 cm. Die Packung in der jeweiligen Rektifikationskolonne 9, 10, 11, 12 war Montz A3-750. Der jeweilige zur Rektifikationskolonne 9, 10, 11, 12 gehörende Kondensator 5 mit Rücklaufteiler für den Rücklauf und den Destillataustrag war selbstverständlich vorhanden, genauso wie die Behälter und Pumpen für die Zudosierung der Edukte, die Behälter und Vorrichtungen für das Destillat, der Behälter für den Sumpfaustrag und der Behälter für die Stabilisierung der Kolonne 9, 10, 11, 12.

Der Dünnschichtverdampfer wurde mit Marlotherm-Öl beheizt.

Die einzelnen Komponenten wurden in diesem Beispiel 4 durch verschiedene Messmethoden ermittelt. Das Wasser wurde hierbei über eine Karl-Fischer Titration bestimmt, wohingegen 2-Octylacrylat, Cyclohexan und 2-Octanol über ein quantitatives GC ermittelt wurde. Die restlichen Komponenten wurden über ein qualitatives GC bestimmt.

Cyclohexan-Abtrennung

Das aus der Wasserwäsche-Extraktionseinheit 8 kommende Gemisch hatte folgende Zusammensetzung:

| | |
|---|---|
| Wasser | 0,30 Gew.-% |
| Cyclohexan | 23,80 Gew.-% |
| Octene | 0,29 Gew.-% |
| 2-Octanol | 3,95 Gew.-% |
| 2-Octylacrylat | 68,80 Gew.-% |
| Diacrylsäureester | 1,14 Gew.-% |
| Oxiester | 1,54 Gew.-% |
| Unbekannte | Rest |

Die Zulaufmenge zum Kopf der Schleppmittel-Rektifikationskolonne 9 betrug 300 g/h.

Der Absolutdruck im Kolonnenkopf betrug 100 mbar und die Sumpftemperatur war 105°C.

Das ablaufende Sumpfprodukt von 230 g/h hatte folgende Zusammensetzung:

| | |
|---|---|
| Wasser | 0,00 Gew.-% |
| Cyclohexan | 1,07 Gew.-% |
| Octene | 0,34 Gew.-% |
| 2-Octanol | 5,14 Gew.-% |
| 2-Octylacrylat | 89,72 Gew.-% |

(fortgesetzt)

| | |
|---|---|
| Diacrylsäureester | 1,49 Gew.-% |
| Oxiester | 2,01 Gew.-% |
| Unbekannte | Rest |

Das ablaufende Destillat von 70 g/h hatte folgende Zusammensetzung:

| | |
|---|---|
| Wasser | 1,28 Gew.-% |
| Cyclohexan | 98,50 Gew.-% |
| Octene | 0,11 Gew.-% |
| 2-Octanol | 0,05 Gew.-% |
| 2-Octylacrylat | 0,06 Gew.-% |
| Diacrylsäureester | 0,00 Gew.-% |
| Oxiester | 0,00 Gew.-% |

2-Octanol-Abtrennung

Für die Abtrennung von 2-Octanol wurde die gleiche Versuchsapparatur wie bei der vorherigen Verfahrensstufe verwendet. Der aus der Cyclohexan-Abtrennung angefallene Sumpfablauf wurde mit einer Menge von 230 g/h am Kopf der 2-Octanol-Rektifikationskolonne 10 zudosiert. Der Absolutdruck im Kolonnenkopf betrug 10 mbar und die Sumpftemperatur war 89°C.

Das ablaufende Sumpfprodukt von 190 g/h hatte folgende Zusammensetzung:

| | |
|---|---|
| Wasser | 0,00 Gew.-% |
| Cyclohexan | 0,00 Gew.-% |
| Octene | 0,00 Gew.-% |
| 2-Octanol | 0,01 Gew.-% |
| 2-Octylacrylat | 95,47 Gew.-% |
| Diacrylsäureester | 1,80 Gew.-% |
| Oxiester | 2,43 Gew.-% |
| Unbekannte | Rest |

Das ablaufende Destillat von 40 g/h hatte folgende Zusammensetzung:

| | |
|---|---|
| Wasser | 0,00 Gew.-% |
| Cyclohexan | 6,12 Gew.-% |
| Octene | 1,98 Gew.-% |
| 2-Octanol | 29,50 Gew.-% |
| 2-Octylacrylat | 62,40 Gew.-% |
| Diacrylsäureester | 0,00 Gew.-% |
| Oxiester | 0,00 Gew.-% |

Reinsieder-Abtrennung

Für die Reinsieder-Abtrennung wurde die gleiche Versuchsapparatur wie bei den vorherigen Verfahrensstufen verwendet. Der aus Versuch der 2-Octanol-Abtrennung angefallene Sumpfablauf wurde mit einer Menge von 190 g/h am Sumpf der Reinsieder-Rektifikationskolonne 11 zudosiert. Der Absolutdruck im Kolonnenkopf betrug 10 mbar und die Sumpftemperatur war 95 °C. Am Rücklaufteiler wurde ein Rücklaufverhältnis von 3 g/g eingestellt. Zur Vermeidung von Polymerisation in der Versuchsapparatur wurde am Kopf der Reinsieder-Rektifikationskolonne 11 mit 2 g/h 2-Octylacrylat (stabilisiert mit 1 % Phenothiazin) zudosiert.

Das ablaufende Sumpfprodukt von 32 g/h hatte folgende Zusammensetzung:

| Wasser | 0,00 Gew.-% |
|---|---|
| Cyclohexan | 0,00 Gew.-% |
| Octene | 0,00 Gew.-% |
| 2-Octanol | 0,00 Gew.-% |
| 2-Octylacrylat | 74,49 Gew.-% |
| Diacrylsäureester | 10,69 Gew.-% |
| Oxiester | 14,44 Gew.-% |
| Phenothiazin | 0,13 Gew.-% |
| Unbekannte | Rest |

Das ablaufende Destillat von 160 g/h hatte folgende Zusammensetzung:

| Wasser | 0,00 Gew.-% |
|---|---|
| Cyclohexan | 0,00 Gew.-% |
| Octene | 0,00 Gew.-% |
| 2-Octanol | 0,01 Gew.-% |
| 2-Octylacrylat | 99,70 Gew.-% |
| Diacrylsäureester | 0,00 Gew.-% |
| Oxiester | 0,00 Gew.-% |
| Unbekannte | Rest |

Schwersieder-Abtrennung

Für die Abtrennung der Schwersieder wurde die gleiche Versuchsapparatur wie bei den vorherigen Verfahrensstufen verwendet. Der aus der Reinsieder-Abtrennung angefallene Sumpfablauf wurde mit einer Menge von 32 g/h am Sumpf der Schwersieder-Rektifikationskolonne 12 zudosiert. Der Absolutdruck im Kolonnenkopf betrug 5 mbar und die Sumpftemperatur war 100°C. Am Rücklaufteiler wurde ein Rücklaufverhältnis von 3 g/g eingestellt.

Das ablaufende Sumpfprodukt von 16 g/h hatte folgende Zusammensetzung:

| Wasser | 0,00 Gew.-% |
|---|---|
| Cyclohexan | 0,00 Gew.-% |
| Octene | 0,00 Gew.-% |
| 2-Octanol | 0,00 Gew.-% |
| 2-Octylacrylat | 49,27 Gew.-% |
| Diacrylsäureester | 21,37 Gew.-% |
| Oxiester | 28,87 Gew.-% |
| Phenothiazin | 0,25 Gew.-% |
| Unbekannte | Rest |

Das ablaufende Destillat von 16 g/h hatte folgende Zusammensetzung:

| Wasser | 0,00 Gew.-% |
|---|---|
| Cyclohexan | 0,00 Gew.-% |
| Octene | 0,00 Gew.-% |
| 2-Octanol | 0,00 Gew.-% |
| 2-Octylacrylat | 99,72 Gew.-% |
| Diacrylsäureester | 0,00 Gew.-% |
| Oxiester | 0,00 Gew.-% |
| Unbekannte | Rest |

**Patentansprüche**

1. Verfahren zur Herstellung von 2-Octyl(meth)acrylat durch Umsetzung von 2-Octanol mit (Meth)acrylsäure in Gegenwart eines sauren Veresterungskatalysators, eines Polymerisationsinhibitors und des Schleppmittels Cyclohexan umfassend die Schritte:

   • Bereitstellen einer Reaktoreinheit (24), wobei sich ein Reaktor (1) mit einem Reaktorheizelement (30) innerhalb der Reaktoreinheit (24) befindet,
   • Zuführen von 2-Octanol (13), (Meth)acrylsäure (14), saurem Veresterungskatalysator (15), Cyclohexan (17) und Polymerisationsinhibitor (31) in den Reaktor (1),
   • Durchführen einer Veresterung in dem Reaktor (1) unter Bildung eines flüssigen Reaktionsgemischs, wobei die Veresterung in dem Reaktor (1) bei einer Sumpftemperatur im Bereich von 90 bis 130 °C und bei einem Absolutdruck im Bereich von 0,5 bis 2,0 bar stattfindet, und ein resultierender Reaktionsaustrag aus dem Reaktor (1) erhalten wird, wobei der resultierende Reaktionsaustrag zumindest 2-Octyl(meth)acrylat, 2-Octanol, (Meth) acrylsäure, sauren Veresterungskatalysator, Cyclohexan, Veresterungswasser und Polymerisationsinhibitor enthält, und das bei der Veresterung entstehende Veresterungswasser zusammen mit dem Schleppmittel Cyclohexan ein heterogenes Azeotrop bildet,
   • Verdampfen des heterogenen Azeotrops aus dem flüssigen Reaktionsgemisch des Reaktors (1), wobei das Verdampfen durch das Reaktorheizelement (30) erreicht wird, und
   • Abtrennen des gasförmigen heterogenen Azeotrops aus dem Reaktor (1),
   wobei das gasförmige heterogene Azeotrop in einem Kondensator (5) kondensiert und anschließend einem Phasenscheider (6) zugeführt wird und das Veresterungswasser als untere Phase und das Cyclohexan als obere Phase in diesem Phasenscheider (6) abgetrennt wird.

2. Verfahren nach Anspruch 1, wobei das gasförmige heterogene Azeotrop eine der Reaktoreinheit (24) nachgeschaltete Azeotrop-Rektifikationskolonneneinheit (25) zugeführt wird, wobei sich eine Azeotrop-Rektifikationskolonne (4) innerhalb der Azeotrop-Rektifikationskolonneneinheit (25) befindet, und die Azeotrop-Rektifikationskolonne (4) bei einem Absolutdruck im Bereich von 0,5 bis 2 bar und bei einer Sumpftemperatur im Bereich von 90 bis 130 °C betrieben wird, und das heterogene Azeotrop über den Kopf der Azeotrop-Rektifikationskolonne (4) abgetrennt, in einem Kondensator (5) kondensiert und anschließend einem Phasenscheider (6) zugeführt wird, wobei im Phasenscheider (6) das Veresterungswasser (16) als untere Phase abgeschieden wird, wohingegen das Cyclohexan als obere Phase im Phasenscheider (6) abgetrennt wird.

3. Verfahren nach Anspruch 1 oder 2, wobei das im Phasenscheider (6) als obere Phase gewonnene Cyclohexan teilweise oder vollständig der Reaktoreinheit (24) zurückgeführt wird.

4. Verfahren nach Anspruch 2, wobei das im Phasenscheider (6) als obere Phase gewonnene Cyclohexan zu einem Gewichtsanteil im Bereich von 40 Gew.-% bis 100 Gew.-%, bevorzugt im Bereich von 50 bis 99,9 Gew.-%, der Reaktoreinheit (24) zurückgeführt wird, und
   der sich ergebende Restanteil des gewonnenen Cyclohexans im Bereich von unterhalb des Kopfs bis zur Mitte der Azeotrop-Rektifikationskolonne (4) zurückgeführt wird.

5. Verfahren nach einem der Ansprüche 1 bis 4, wobei im Anschluss an die Veresterung eine alkalische Extraktion des resultierenden Reaktionsaustrags mittels einer alkalischen Lösung (19) erfolgt,

   indem der saure Veresterungskatalysator sowie nicht umgesetzte (Meth)acrylsäure in einer Neutralisations-Extraktionseinheit (7) neutralisiert werden, und
   sich dadurch eine obere Neutralisations-Phase, welche 2-Octyl(meth)acrylat enthält, und eine untere Neutralisations-Phase, welche durch die Neutralisation erzeugte Salze sowie Wasser (20) enthält, ergeben.

6. Verfahren nach Anspruch 5, wobei im Anschluss an die alkalische Extraktion des Reaktionsaustrags eine Extraktion der aus der Neutralisations-Extraktionseinheit (7) gewonnenen oberen Neutralisations-Phase mit Wasser (18) in einer Wasserwäsche-Extraktionseinheit (8) erfolgt,
   wobei eine mit Wasser und Reste von Salzen enthaltende untere Wasserwäsche-Phase und eine mit 2-Octyl(meth) acrylat enthaltende obere Wasserwäsche-Phase gebildet werden.

7. Verfahren nach Anspruch 5 oder 6, wobei im Anschluss an die alkalische Extraktion des Reaktionsaustrags oder im Anschluss an die Wasserwäsche-Extraktion eine Abtrennung des Schleppmittels Cyclohexan aus der oberen Neutralisations-Phase oder der oberen Wasserwäsche-Phase in einer Schleppmittel-Rektifikationskolonneneinheit erfolgt,

wobei sich eine Schleppmittel-Rektifikationskolonne (9) innerhalb der Schleppmittel-Rektifikationskolonneneinheit befindet, und

die Schleppmittel-Rektifikationskolonne (9) bei einem verminderten Absolutdruck im Bereich von 0,05 bis 0,9 bar und bei einer Sumpftemperatur im Bereich von 70 bis 120 °C betrieben wird, und über den Kopf der Schleppmittel-Rektifikationskolonne (9) eine organische Phase abgezogen wird, die das Schleppmittel Cyclohexan, Octene, 2-Octanol sowie einen Massenstromanteil an 2-Octyl(meth)acrylat im Bereich von 0,1 bis 5,0 %, bezogen auf die zugeführte obere Neutralisations-Phase oder die zugeführte obere Wasserwäsche-Phase, enthält,

wobei die über Kopf abgeführten Komponenten einen Schleppmittel-Massenstrom bilden, dieser kondensiert wird und im Bereich von 50 bis 100 % des Reaktors (1) der Reaktoreinheit (24) zugeführt wird,

wobei der restliche Schleppmittel-Massenstromanteil kondensiert und am Kopf der Schleppmittel-Rektifikationskolonne (9) zurückgeführt wird, wohingegen Schwersieder, welche Di(meth)acrylsäureester und Oxiester, wie den Alkoxialkylester der (Meth)acrylsäure, enthalten, sowie 2-Octanol und 2-Octyl(meth)acrylat durch einen Sumpfablauf der Schleppmittel-Rektifikationskolonne (9) abgezogen werden,

wobei im Bereich von 20 bis 95 % des Massenstroms des Sumpfablaufs durch einen Verdampfer strömt und anschließend in die Schleppmittel-Rektifikationskolonne (9) zurückgeführt wird.

8.  Verfahren nach Anspruch 5 oder 6, wobei das 2-Octyl(meth)acrylat in einer Trennwandkolonne (131) von den Cyclohexan und 2-Octanol enthaltenden restlichen Komponenten abgetrennt wird.

9.  Verfahren nach Anspruch 7, wobei im Anschluss an die Abtrennung des Schleppmittels Cyclohexan eine Abtrennung des 2-Octanols aus dem durch den Sumpfablauf der Schleppmittel-Rektifikationskolonne (9) gegebenen Massenstrom in einer 2-Octanol-Rektifikationskolonneneinheit oder einer 2-Octanol Verdampfereinheit erfolgt,

wobei sich eine 2-Octanol-Rektifikationskolonne (10) innerhalb der 2-Octanol-Rektifikationskolonneneinheit oder ein 2-Octanol-Verdampfer (110) innerhalb der 2-Octanol-Verdampfereinheit befindet, und die 2-Octanol-Rektifikationskolonne (10) oder der 2-Octanol-Verdampfer (110) bei einem verminderten Absolutdruck im Bereich von 0,005 bis 0,10 bar und bei einer Sumpftemperatur im Bereich von 70 bis 130 °C betrieben wird, und der durch den Sumpfablauf der Schleppmittel-Rektifikationskolonne (9) gegebene Massenstrom im Bereich von unterhalb des Kopfs bis zur Mitte der 2-Octanol-Rektifikationskolonne (10) oder an einem 2-Octanol Einlass des 2-Octanol-Verdampfers (110) zudosiert wird, und die über den Kopf der 2-Octanol-Rektifikationskolonne (10) oder über einen 2-Octanol-Verdampfer Auslass des 2-Octanol-Verdampfers (110) abgeführten Komponenten im Bereich von 20 bis 50 %, bezogen auf den Massenstrom der abgeführten Komponenten, der Reaktoreinheit (24) zugeführt werden,

wobei die abgeführten Komponenten im Bereich von 2 bis 40 Gew.-% aus 2-Octanol bestehen, wohingegen 2-Octyl(meth)acrylat und Schwersieder, die Di(meth)acrylsäureester und Oxiester enthalten, durch einen Sumpfablauf der 2-Octanol-Rektifikationskolonne (10) oder des 2-Octanol-Verdampfers (110) abgezogen werden,

wobei 20 bis 95 % des Massenstroms des Sumpfablaufs durch einen Verdampfer strömt und anschließend in die 2-Octanol-Rektifikationskolonne (10) oder in den 2-Octanol-Verdampfer (110) zurückgeführt wird.

10.  Verfahren nach Anspruch 9, wobei im Anschluss an die Abtrennung des 2-Octanols eine Reinsieder-Abtrennung aus dem durch den Sumpfablauf der 2-Octanol-Rektifikationskolonne (10) oder des 2-Octanol-Verdampfers (110) gegebenen Massenstrom in einer Reinsieder-Rektifikationskolonneneinheit oder einer Reinsieder-Verdampfereinheit erfolgt, wobei sich eine Reinsieder-Rektifikationskolonne (11) innerhalb der Reinsieder-Rektifikationskolonneneinheit oder ein Reinsieder-Verdampfer (111) innerhalb der Reinsieder-Verdampfereinheit befindet, und

die Reinsieder-Rektifikationskolonne (11) oder der Reinsieder-Verdampfer (111) bei einem verminderten Absolutdruck im Bereich von 0,002 bis 0,05 bar und bei einer Sumpftemperatur im Bereich von 80 bis 130 °C betrieben wird, und

der durch den Sumpfablauf der 2-Octanol-Rektifikationskolonne (10) oder des 2-Octanol-Verdampfers (110) gegebene Massenstrom im Bereich von unterhalb des Kopfs bis zur Mitte der Reinsieder-Rektifikationskolonne (11) oder an einem Reinsieder-Verdampfer Einlass des Reinsieder-Verdampfers (111) zudosiert wird, und zur Stabilisierung der Reinsieder-Rektifikationskolonne (11) oder dem Reinsieder-Verdampfer (111) 2-Octyl(meth)acrylat sowie einen Polymerisationsinhibitor jeweils im Bereich von 0,01 bis 1,0 %, bezogen auf den durch den Sumpfablauf der 2-Octanol-Rektifikationskolonne (10) oder den durch den Sumpfablauf des 2-Octanol-Verdampfers (110) gegebenen Massenstrom, im Bereich vom Sumpf bis zur Mitte der Reinsieder-Rektifikationskolonne (11) oder an einem Reinsieder-Verdampfer Einlass des Reinsieder-Verdampfers (111) zudosiert werden, und

das 2-Octyl(meth)acrylat (21) über den Kopf der Reinsieder-Rektifikationskolonne (11) oder über einen Reinsieder-Verdampfer Auslass des Reinsieder-Verdampfers (111) abgezogen wird, wohingegen Schwersieder, die Di(meth)acrylsäureester und Oxiester, wie den Alkoxialkylester der (Meth)acrylsäure, enthalten durch einen Sumpfablauf der Reinsieder-Rektifikationskolonne (11) oder des Reinsieder-Verdampfers (111) abgezogen werden.

11. Verfahren nach Anspruch 10, wobei im Anschluss an die Reinsieder-Abtrennung eine Schwersieder-Abtrennung aus dem durch den Sumpfablauf der Reinsieder-Rektifikationskolonne (11) oder des Reinsieder-Verdampfers (111) gegebenen Massenstrom in einer Schwersieder-Rektifikationskolonneneinheit oder einer Schwersieder-Verdampfereinheit erfolgt,

wobei sich eine Schwersieder-Rektifikationskolonne (12) innerhalb der Schwersieder-Rektifikationskolonneneinheit oder ein Schwersieder-Verdampfer (112) innerhalb der Schwersieder-Verdampfereinheit befindet, und die Schwersieder-Rektifikationskolonne (12) oder der Schwersieder-Verdampfer (112) bei einem verminderten Absolutdruck im Bereich von 0,002 bis 0,05 bar und bei einer Sumpftemperatur im Bereich von 80 bis 150 °C betrieben wird, und

der durch den Sumpfablauf der Reinsieder-Rektifikationskolonne (11) oder dem Reinsieder-Verdampfers (111) gegebene Massenstrom im Bereich vom Sumpf bis zur Mitte der Schwersieder-Rektifikationskolonne (12) oder an einen Schwersieder-Verdampfer Einlass des Schwersieder-Verdampfers (112) zudosiert wird, und

der über den Kopf der Schwersieder-Rektifikationskolonne (12) oder des Schwersieder-Verdampfers (112) entnommene Abzug kondensiert wird und im Bereich vom Sumpf bis zur Mitte der Reinsieder-Rektifikationskolonne (11) oder am Reinsieder-Verdampfer Einlass des Reinsieder-Verdampfers (111) zugeführt wird.

12. Verfahren nach einem der vorhergehenden Ansprüche, wobei die Sumpftemperatur in dem Reaktor (1) der Reaktoreinheit (24) im Bereich von 100 bis 125 °C, bevorzugt im Bereich von 110 bis 115 °C, beträgt.

13. Verfahren nach einem der vorhergehenden Ansprüchen, wobei dem Reaktor (1) der Reaktoreinheit (24) Cyclohexan in einer Menge im Bereich von 100 bis 600 Gew.-%, bevorzugt im Bereich von 200 bis 500 Gew.-%, besonders bevorzugt im Bereich von 350 bis 450 Gew.-%, bezogen auf die dem Reaktor (1) der Reaktoreinheit (24) zugeführten Massenmengen an 2-Octanol (13) und (Meth)acrylsäure (14), zugeführt wird.

14. Verfahren nach einem der vorhergehenden Ansprüchen, wobei der Katalysator-Anteil in dem Reaktor (1) der Reaktoreinheit (24) maximal 10 Gew.-%, bezogen auf die Summe der in dem Reaktor (1) der Reaktoreinheit (24) vorhandenen Komponenten an 2-Octanol und (Meth)acrylsäure, beträgt.

15. Verfahren nach einem der vorhergehenden Ansprüchen, wobei die ohne das Schleppmittel Cyclohexan in den Reaktor (1) der Reaktoreinheit (24) zufließenden Komponenten folgende Gewichtsanteile aufweisen:

| | | | | | |
|---|---|---|---|---|---|
| • | 2-Octanol: | 40,00 | bis | 84,39 | Gew.-% |
| • | (Meth)acrylsäure: | 15,00 | bis | 59,39 | Gew.-% |
| • | saurer Veresterungskatalysator: | 0,50 | bis | 10,00 | Gew.-% |
| • | Polymerisationsinhibitor: | 0,01 | bis | 1,00 | Gew.-% |
| • | Restliche Komponenten: | 0,10 | bis | 5,00 | Gew.-%, |

und das Schleppmittel Cyclohexan in einer solchen Menge so zudosiert wird, dass in dem Reaktor (1) der Reaktoreinheit (24) eine Konzentration an Cyclohexan im Bereich von 10 bis 90 Gew.-% entsteht, wobei sich diese Angabe des Gew.-%-Anteils auf die in dem Reaktor (1) der Reaktoreinheit (24) vorhandenen Komponenten inklusive dem Cyclohexan bezieht,

und/oder die ohne das Schleppmittel Cyclohexan und ohne das Veresterungswasser aus dem Reaktor (1) der Reaktoreinheit (24) insgesamt herausströmenden Komponenten, nämlich den resultierenden, flüssigen Reaktionsaustrag plus den aus dem Reaktionsgemisch verdampften und aus der Reaktoreinheit (24) abgeführten Anteil, folgende Gewichtsanteile aufweisen:

| | | | | | |
|---|---|---|---|---|---|
| • | 2-Octyl(meth)acrylat: | 50,00 | bis | 95,00 | Gew.-% |

(fortgesetzt)

| | | | | | | |
|---|---|---|---|---|---|---|
| • | 2-Octanol: | 1,00 | bis | 30,00 | Gew.-% |
| • | (Meth)acrylsäure: | 1,00 | bis | 15,00 | Gew.-% |
| • | saurer Veresterungskatalysator: | 0,50 | bis | 10,00 | Gew.-% |
| • | Polymerisationsinhibitor: | 0,01 | bis | 1,00 | Gew.-% |
| • | Restliche Komponenten: | 2,49 | bis | 10,00 | Gew.-%, |

und das sich durch das Cyclohexan sowie das Veresterungswasser bildende heterogene Azeotrop in einer Konzentration im Bereich von 10 bis 50 Gew.-% aus dem Reaktor (1) der Reaktoreinheit (24) herausströmt, wobei sich diese Angabe des Gew.-%-Anteils auf die insgesamt herausströmenden Komponenten inklusive des Cyclohexans und des Veresterungswassers bezieht.

**Claims**

1. A process for producing 2-octyl (meth)acrylate by reacting 2-octanol with (meth)acrylic acid in the presence of an acidic esterification catalyst, a polymerization inhibitor and the azeotroping agent cyclohexane, comprising the steps of:

   • providing a reactor unit (24), wherein a reactor (1) having a reactor heating element (30) is located within the reactor unit (24),
   • feeding 2-octanol (13), (meth)acrylic acid (14), acidic esterification catalyst (15), cyclohexane (17) and polymerization inhibitor (31) into the reactor (1),
   • carrying out an esterification in the reactor (1) to form a liquid reaction mixture, wherein the esterification in the reactor (1) is conducted at a bottom temperature in the range of 90 to 130°C and at an absolute pressure in the range of 0.5 to 2.0 bar, and a resulting reaction discharge from the reactor (1) is obtained,
   wherein the resulting reaction discharge comprises at least 2-octyl (meth)acrylate, 2-octanol, (meth)acrylic acid, acidic esterification catalyst, cyclohexane, water of esterification and polymerization inhibitor, and the water of esterification formed in the esterification together with the azeotroping agent cyclohexane forms a heterogeneous azeotrope,
   • evaporating the heterogeneous azeotrope from the liquid reaction mixture of the reactor (1), wherein the evaporation is accomplished by the reactor heating element (30), and
   • removing the gaseous heterogeneous azeotrope from the reactor (1),

   wherein the gaseous heterogeneous azeotrope is condensed in a condenser (5) and is then fed to a phase separator (6) and the water of esterification is separated off as the lower phase and the cyclohexane as upper phase in this phase separator (6).

2. The process according to claim 1, wherein the gaseous heterogeneous azeotrope is fed to an azeotrope rectification column unit (25) downstream of the reactor unit (24),

   wherein an azeotrope rectification column (4) is located within the azeotrope rectification column unit (25), and the azeotrope rectification column (4) is operated at an absolute pressure in the range of 0.5 to 2 bar and at a bottom temperature in the range of 90 to 130°C, and the heterogeneous azeotrope is removed via the top of the azeotrope rectification column (4), is condensed in a condenser (5) and is then fed to a phase separator (6),
   wherein the water of esterification (16) is separated off as the lower phase in the phase separator (6), while the cyclohexane is separated off as the upper phase in the phase separator (6).

3. The process according to claim 1 or 2, wherein the cyclohexane obtained as upper phase in the phase separator (6) is partially or fully recycled to the reactor unit (24).

4. The process according to claim 2, wherein the cyclohexane obtained as upper phase in the phase separator (6) is recycled to the reactor unit (24) at a proportion by weight in the range of 40% by weight to 100% by weight, preferably in the range of 50 to 99.9% by weight, and
   the resulting residual proportion of the cyclohexane obtained is recycled into the area from below the top to the middle of the azeotrope rectification column (4).

5. The process according to any of claims 1 to 4, wherein subsequent to the esterification, an alkaline extraction of the resulting reaction discharge is carried out by means of an alkaline solution (19),

in which the acidic esterification catalyst and unreacted (meth)acrylic acid are neutralized in a neutralization extraction unit (7), and
which results in an upper neutralization phase comprising 2-octyl (meth)acrylate, and a lower neutralization phase comprising salts generated by the neutralization and water (20).

6. The process according to claim 5, wherein subsequent to the alkaline extraction of the reaction discharge, the upper neutralization phase obtained from the neutralization extraction unit (7) is extracted with water (18) in a waterwash extraction unit (8),
wherein a lower waterwash phase comprising water and salt residues and an upper waterwash phase comprising 2-octyl (meth)acrylate are formed.

7. The process according to claim 5 or 6, wherein subsequent to the alkaline extraction of the reaction discharge or subsequent to the waterwash extraction, the azeotroping agent cyclohexane is separated off from the upper neutralization phase or from the upper waterwash phase in an azeotrope rectification column unit,

wherein an azeotrope rectification column (9) is located within the azeotrope rectification column unit, and
the azeotrope rectification column (9) is operated at a reduced absolute pressure in the range of 0.05 to 0.9 bar and at a bottom temperature in the range of 70 to 120°C, and an organic phase is removed via the top of the azeotrope rectification column (9) comprising the azeotroping agent cyclohexane, octenes, 2-octanol and a mass flow proportion of 2-octyl (meth)acrylate in the range of 0.1 to 5.0%, based on the upper neutralization phase fed in or the upper waterwash phase fed in,
wherein the components discharged overhead form an azeotrope mass flow, this being condensed and fed into the reactor (1) of the reactor unit (24) in the range of 50 to 100%,
wherein the residual azeotrope mass flow proportion is condensed and is recycled to the top of the azeotrope rectification column (9), while high boilers comprising di(meth)acrylic acid esters and oxy esters, such as the alkoxyalkyl esters of (meth)acrylic acid, and also 2-octanol and 2-octyl (meth)acrylate are removed through a bottom discharge of the azeotrope rectification column (9),
wherein in the range of 20 to 95% of the mass flow of the bottom discharge flows through an evaporator and is subsequently recycled to the azeotrope rectification column (9).

8. The process according to claim 5 or 6, wherein the 2-octyl (meth)acrylate is separated in a dividing wall column (131) from the residual components comprising cyclohexane and 2-octanol.

9. The process according to claim 7, wherein subsequent to the removal of the azeotroping agent cyclohexane, 2-octanol is separated off from the mass flow produced by the bottom discharge of the azeotrope rectification column (9) in a 2-octanol rectification column unit or a 2-octanol evaporation unit,

wherein a 2-octanol rectification column (10) is located within the 2-octanol rectification column unit or a 2-octanol evaporator (110) is located within the 2-octanol evaporation unit, and the 2-octanol rectification column (10) or the 2-octanol evaporator (110) is operated at a reduced absolute pressure in the range of 0.005 to 0.10 bar and at a bottom temperature in the range of 70 to 130°C, and
the mass flow produced by the bottom discharge of the azeotrope rectification column (9) is metered in to the area below the head to the middle of the 2-octanol rectification column (10) or to a 2-octanol inlet of the 2-octanol evaporator (110), and
the components discharged via the head of the 2-octanol rectification column (10) or via a 2-octanol evaporator outlet of the 2-octanol evaporator (110) are fed to the reactor unit (24) in the range of 20 to 50%, based on the mass flow of the discharged components,
wherein the discharged components in the range of 2 to 40% by weight consist of 2-octanol, while 2-octyl (meth)acrylate and high boilers comprising di(meth)acrylic acid esters and oxy esters are withdrawn through a bottom discharge of the 2-octanol rectification column (10) or the 2-octanol evaporator (110),
wherein 20 to 95% of the mass flow of the bottom discharge flows through an evaporator and is subsequently recycled to the 2-octanol rectification column (10) or to the 2-octanol evaporator (110).

10. The process according to claim 9, wherein subsequent to the separation of 2-octanol, a pure boiler separation from the mass flow provided through the bottom discharge of the 2-octanol rectification column (10) or the 2-octanol evaporator (110) is carried out in a pure boiler rectification column unit or a pure boiler evaporation unit,

wherein a pure boiler rectification column (11) is located within the pure boiler rectification column unit or a pure boiler evaporator (111) within the pure boiler evaporation unit, and

the pure boiler rectification column (11) or the pure boiler evaporator (111) is operated at a reduced absolute pressure in the range of 0.002 to 0.05 bar and at a bottom temperature in the range of 80 to 130°C, and

the mass flow produced by the bottom discharge of the 2-octanol rectification column (10) or the 2-octanol evaporator (110) is metered into the area from below the head to the middle of the pure boiler rectification column (11) or to a pure boiler evaporator inlet of the pure boiler evaporator (111), and

to stabilize the pure boiler rectification column (11) or the pure boiler evaporator (111), 2-octyl (meth)acrylate and a polymerisation inhibitor, each in the range of 0.01 to 1.0 %, based on the mass flow produced by the bottom discharge of the 2-octanol rectification column (10) or by the bottom discharge of the 2-octanol evaporator (110), are metered into the area from the bottom up to the middle of the pure boiler rectification column (11) or to a pure boiler evaporator inlet of the pure boiler evaporator (111), and

the 2-octyl (meth)acrylate (21) is withdrawn via the head of the pure boiler rectification column (11) or via a pure boiler evaporator outlet of the pure boiler evaporator (111), while high boilers comprising di(meth)acrylic acid esters and oxy esters, such as the alkoxyalkyl esters of (meth)acrylic acid, are withdrawn through a bottom discharge of the pure boiler rectification column (11) or of the pure boiler evaporator (111).

11. The process according to claim 10, wherein subsequent to the pure boiler separation, a high boiler separation from the mass flow produced through the bottom discharge of the pure boiler rectification column (11) or of the pure boiler evaporator (111) is carried out in a high boiler rectification column unit or a high boiler evaporation unit,

wherein a high boiler rectification column (12) is located within the high boiler rectification column unit or a high boiler evaporator (112) within the high boiler evaporator unit, and

the high boiler rectification column (12) or the high boiler evaporator (112) is operated at a reduced absolute pressure in the range of 0.002 to 0.05 bar and at a bottom temperature in the range of 80 to 150°C, and

the mass flow produced by the bottom discharge of the pure boiler rectification column (11) or the pure boiler evaporator (111) is metered into the area from the bottom up to the middle of the high boiler rectification column (12) or to a high boiler evaporator inlet of the high boiler evaporator (112), and

the takeoff withdrawn via the top of the high boiler rectification column (12) or the high boiler evaporator (112) is condensed and is fed into the area from the bottom up to the middle of the pure boiler rectification column (11) or to the pure boiler evaporator inlet of the pure boiler evaporator (111).

12. The process according to any of the preceding claims, wherein the bottom temperature in the reactor (1) of the reactor unit (24) is in the range of 100 to 125°C, preferably in the range of 110 to 115°C.

13. The process according to any of the preceding claims, wherein cyclohexane is fed to the reactor (1) of the reactor unit (24) in an amount in the range of 100 to 600% by weight, preferably in the range of 200 to 500% by weight, particularly preferably in the range of 350 to 450% by weight, based on the amounts by mass of 2-octanol (13) and (meth) acrylic acid (14) fed to the reactor (1) of the reactor unit (24).

14. The process according to any of the preceding claims, wherein the proportion of catalyst in the reactor (1) of the reactor unit (24) is at maximum 10% by weight, based on the sum of the components of 2-octanol and (meth)acrylic acid present in the reactor (1) of the reactor unit (24).

15. The process according to any of the preceding claims, wherein the components flowing into the reactor (1) of the reactor unit (24) without the azeotroping agent cyclohexane have the following proportions by weight:

• 2-octanol: 40.00% to 84.39% by weight
• (meth)acrylic acid: 15.00% to 59.39% by weight
• acidic esterification catalyst: 0.50% to 10.00% by weight
• polymerization inhibitor: 0.01% to 1.00% by weight
• residual components: 0.10% to 5.00% by weight, and the azeotroping agent cyclohexane is added in an amount such that a concentration of cyclohexane forms in the reactor (1) of the reactor unit (24) in the range of 10 to 90% by weight, where these figures of the percentage by weight relate to the components present in the reactor (1) of the reactor unit (24) including the cyclohexane, and/or without the azeotroping agent cyclohexane and without the water of esterification, the total components flowing out of the reactor (1) of the reactor unit (24), namely the resulting liquid reaction discharge plus the portion evaporated from the reaction mixture and discharged from the reactor unit (24) have the following proportions by weight:
- 2-octyl (meth)acrylate: 50.00% to 95.00% by weight
- 2-octanol: 1.00% to 30.00% by weight
- (meth)acrylic acid: 1.00% to 15.00% by weight
- acidic esterification catalyst: 0.50% to 10.00% by weight
- polymerization inhibitor: 0.01% to 1.00% by weight
- residual components: 2.49% to 10.00% by weight,

and the heterogeneous azeotrope formed by the cyclohexane and the water of esterification flows out of the reactor (1) of the reactor unit (24) at a concentration in the range of 10 to 50% by weight, where these figures of the percentage by weight relate to the total components flowing out including the cyclohexane and the water of esterification.

## Revendications

1. Procédé pour la préparation de (méth)acrylate de 2-octyle par mise en réaction de 2-octanol avec de l'acide (méth) acrylique en présence d'un catalyseur d'estérification acide, d'un inhibiteur de polymérisation et de l'agent d'entraînement cyclohexane, comprenant les étapes :

   • mise à disposition d'une unité de réacteur (24), dans laquelle un réacteur (1) comportant un élément chauffant (30) de réacteur se trouve à l'intérieur de l'unité de réacteur (24),
   • introduction de 2-octanol (13), d'acide (méth)acrylique (14), d'un catalyseur d'estérification acide (15), de cyclohexane (17) et d'un inhibiteur de polymérisation (31) dans le réacteur (1),
   • exécution d'une estérification dans le réacteur (1) avec formation d'un mélange réactionnel liquide, l'estérification ayant lieu dans le réacteur (1) à une température de fond de réacteur dans la plage de 90 à 130 °C et sous une pression absolue dans la plage de 0,5 à 2,0 bar, et un courant résultant issu de la réaction est obtenu à partir du réacteur (1),
   le courant résultant issu de la réaction contenant au moins du (méth)acrylate de 2-octyle, du 2-octanol, de l'acide (méth)acrylique, du catalyseur d'estérification acide, du cyclohexane, de l'eau d'estérification et de l'inhibiteur de polymérisation, et l'eau d'estérification produite lors de l'estérification formant conjointement avec l'agent d'entraînement cyclohexane un azéotrope hétérogène,
   • évaporation de l'azéotrope hétérogène à partir du mélange réactionnel liquide du réacteur (1), l'évaporation étant réalisée par l'élément chauffant (30) de réacteur, et
   • séparation de l'azéotrope hétérogène gazeux à partir du réacteur (1),

   l'azéotrope hétérogène gazeux étant condensé dans un condenseur (5) et étant ensuite envoyé à un séparateur de phases (6) et l'eau d'estérification étant séparée en tant que phase inférieure et le cyclohexane étant séparé en tant que phase supérieure dans ce séparateur de phases (6).

2. Procédé selon la revendication 1, dans lequel l'azéotrope hétérogène gazeux est envoyé à une unité de colonne de rectification d'azéotrope (25) raccordée en aval à l'unité de réacteur (24),

   dans lequel une colonne de rectification d'azéotrope (4) se trouve à l'intérieur de l'unité de colonne de rectification d'azéotrope (25), et la colonne de rectification d'azéotrope (4) est utilisée sous une pression absolue dans la plage de 0,5 à 2 bar et à une température de pied dans la plage de 90 à 130 °C, et l'azéotrope hétérogène est séparé par la tête de la colonne de rectification d'azéotrope (4), condensé dans un condenseur (5) et ensuite envoyé à un séparateur de phases (6),
   l'eau d'estérification (16) étant séparée en tant que phase inférieure dans le séparateur de phases (6), tandis que le cyclohexane est séparé en tant que phase supérieure dans le séparateur de phases (6).

3. Procédé selon la revendication 1 ou 2, dans lequel le cyclohexane récupéré en tant que phase supérieure dans le séparateur de phases (6) est partiellement ou totalement renvoyé à l'unité de réacteur (24).

4. Procédé selon la revendication 2, dans lequel le cyclohexane obtenu en tant que phase supérieure dans le séparateur de phases (6) est renvoyé à l'unité de réacteur (24) en une proportion en poids dans la plage de 40 % en poids à 100 % en poids, de préférence dans la plage de 50 à 99,9 % en poids, et
   la fraction résiduelle résultante du cyclohexane obtenu est renvoyée dans la zone s'étendant depuis au-dessous de la tête jusqu'au milieu de la colonne de rectification d'azéotrope (4).

5. Procédé selon l'une quelconque des revendications 1 à 4, dans lequel à la suite de l'estérification a lieu une extraction alcaline du courant résultant issu de la réaction, au moyen d'une solution alcaline (19),

> par le fait que le catalyseur d'estérification acide ainsi que l'acide (méth)acrylique n'ayant pas réagi sont neutralisés dans une unité de neutralisation-extraction (7), et
> il en résulte une phase supérieure de neutralisation qui contient du (méth)acrylate de 2-octyle et une phase inférieure de neutralisation qui contient des sels produits par la neutralisation ainsi que de l'eau (20).

6. Procédé selon la revendication 5, dans lequel, à la suite de l'extraction alcaline du courant issu de la réaction a lieu une extraction de la phase supérieure de neutralisation obtenue à partir de l'unité d'extraction de neutralisation (7), avec de l'eau (18) dans une unité d'extraction par lavage à l'eau (8), dans laquelle sont formées une phase inférieure de lavage à l'eau contenant de l'eau et des résidus de sels et une phase supérieure de lavage à l'eau contenant du (méth)acrylate de 2-octyle.

7. Procédé selon la revendication 5 ou 6, dans lequel, à la suite de l'extraction alcaline du courant issu de la réaction ou à la suite de l'extraction par lavage à l'eau a lieu une séparation de l'agent d'entraînement cyclohexane à partir de la phase supérieure de neutralisation ou de la phase supérieure de lavage à l'eau dans une unité de colonne de rectification d'agent d'entraînement,

> dans lequel une colonne de rectification d'agent d'entraînement (9) se trouve à l'intérieur de l'unité de colonne de rectification d'agent d'entraînement, et
> la colonne de rectification d'agent d'entraînement (9) est utilisée sous une pression absolue réduite dans la plage de 0,05 à 0,9 bar et à une température de pied dans la plage de 70 à 120 °C, et par la tête de la colonne de rectification d'agent d'entraînement (9) est évacuée une phase organique qui contient l'agent d'entraînement cyclohexane, de l'octène, du 2-octanol ainsi qu'une proportion de courant massique de (méth)acrylate de 2-octyle dans la plage de 0,1 à 5,0 %, par rapport à la phase supérieure de neutralisation introduite ou à la phase supérieure de lavage à l'eau introduite,
> dans lequel les composants évacués par la tête forment un courant massique d'agent d'entraînement, ce dernier est condensé et envoyé à l'unité de réacteur (24) dans la plage de 50 à 100 % du réacteur (1),
> dans lequel la fraction résiduelle de courant massique d'agent d'entraînement est condensée et renvoyée à la tête de la colonne de rectification d'agent d'entraînement (9), tandis que les composants à haut point d'ébullition, qui contiennent des esters d'acide di(méth)acrylique et des oxyesters, tels que les esters alcoxyalkyliques de l'acide (méth)acrylique, ainsi que du 2-octanol et du (méth)acrylate de 2-octyle, sont évacués par une sortie de pied de la colonne de rectification d'agent d'entraînement (9),
> dans la plage de 20 à 95 % du courant massique de la sortie de pied passant à travers un évaporateur et étant ensuite recyclés dans la colonne de rectification d'agent d'entraînement (9).

8. Procédé selon la revendication 5 ou 6, dans lequel le (méth)acrylate de 2-octyle est séparé d'avec les composants restants contenant du cyclohexane et du 2-octanol dans une colonne à paroi de séparation (131).

9. Procédé selon la revendication 7, dans lequel à la suite de la séparation de l'agent d'entraînement cyclohexane a lieu une séparation du 2-octanol à partir du courant massique fourni par la sortie de pied de la colonne de rectification d'agent d'entraînement (9), dans une unité de colonne de rectification de 2-octanol ou une unité d'évaporation de 2-octanol,

> dans lequel une colonne de rectification de 2-octanol (10) se trouve à l'intérieur de l'unité de colonne de rectification de 2-octanol ou un évaporateur de 2-octanol (110) se trouve à l'intérieur de l'unité d'évaporation de 2-octanol, et la colonne de rectification de 2-octanol (10) ou l'évaporateur de 2-octanol (110) est utilisé(e) sous une pression absolue réduite dans la plage de 0,005 à 0,10 bar et à une température de pied dans la plage de 70 à 130 °C, et
> le courant massique fourni par la sortie de pied de la colonne de rectification d'agent d'entraînement (9) est introduit dans la zone s'étendant depuis au-dessous de la tête jusqu'au milieu de la colonne de rectification de 2-octanol (10) ou à une entrée de 2-octanol de l'évaporateur de 2-octanol (110), et
> les composants évacués par la tête de la colonne de rectification de 2-octanol (10) ou par une sortie d'évaporateur de 2-octanol de l'évaporateur de 2-octanol (110) sont envoyés à l'unité de réacteur (24) dans la plage de 20 à 50 %, par rapport au courant massique des composants évacués,

les composants évacués consistant dans la plage de 2 à 40 % en poids en 2-octanol, tandis que du (méth)acrylate de 2-octyle et des composants à haut point d'ébullition, qui contiennent des esters d'acide di(méth)acrylique et des oxyesters, sont évacués par une sortie de pied de la colonne de rectification de 2-octanol (10) ou de l'évaporateur de 2-octanol (110),

dans la plage de 20 à 95 % du courant massique de la sortie de pied passant à travers un évaporateur et étant ensuite recyclés dans la colonne de rectification de 2-octanol (10) ou dans l'évaporateur de 2-octanol (110).

10. Procédé selon la revendication 9, dans lequel à la suite de la séparation du 2-octanol a lieu une séparation de composants purs à partir du courant massique fourni par la sortie de pied de la colonne de rectification de 2-octanol (10) ou de l'évaporateur de 2-octanol (110) dans une unité de colonne de rectification de composants purs ou une unité d'évaporation de composants purs,

dans lequel une colonne de rectification de composants purs (11) se trouve à l'intérieur de l'unité de colonne de rectification de composants purs ou un évaporateur de composants purs (111) se trouve à l'intérieur de l'unité d'évaporation de composants purs, et

la colonne de rectification de composants purs (11) ou l'évaporateur de composants purs (111) est utilisé(e) sous une pression absolue réduite dans la plage de 0,002 à 0,05 bar et à une température de pied dans la plage de 80 à 130 °C, et

le courant massique fourni par la sortie de pied de la colonne de rectification de 2-octanol (10) ou de l'évaporateur de 2-octanol (110) est introduit dans la zone s'étendant depuis au-dessous de la tête jusqu'au milieu de la colonne de rectification de composants purs (11) ou dans une entrée d'évaporateur de composants purs de l'évaporateur de composants purs (111), et

pour la stabilisation de la colonne de rectification de composants purs (11) ou de l'évaporateur de composants purs (111), du (méth)acrylate de 2-octyle ainsi qu'un inhibiteur de polymérisation sont introduits, chacun dans la plage de 0,01 à 1,0 %, par rapport au courant massique fourni par la sortie de pied de la colonne de rectification de 2-octanol (10) ou au courant massique fourni par la sortie de pied de l'évaporateur de 2-octanol (110), dans la zone s'étendant depuis le pied jusqu'au milieu de la colonne de rectification de composants purs (11) ou à une entrée d'évaporateur de composants purs (111), et

le (méth)acrylate de 2-octyle (21) est évacué par la tête de la colonne de rectification de composants purs (11) ou par une sortie d'évaporateur de composants purs de l'évaporateur de composants purs (111), tandis que les composants à haut point d'ébullition, qui contiennent des esters d'acide di(méth)acrylique et des oxyesters, tels que les esters alcoxyalkyliques de l'acide (méth)acrylique, sont évacués par une sortie de pied de la colonne de rectification de composants purs (11) ou de l'évaporateur de composants purs (111).

11. Procédé selon la revendication 10, dans lequel, à la suite de la séparation de composants purs a lieu une séparation de composants à haut point d'ébullition à partir du courant massique fourni par la sortie de pied de la colonne de rectification de composants purs (11) ou du courant massique fourni par l'évaporateur de composants purs (111), dans une unité de colonne de rectification de composants à haut point d'ébullition ou une unité d'évaporateur de composants à haut point d'ébullition,

dans lequel une colonne de rectification de composants à haut point d'ébullition (12) se trouve à l'intérieur de l'unité de colonne de rectification de composants à haut point d'ébullition ou un évaporateur de composants à haut point d'ébullition (112) se trouve à l'intérieur de l'unité d'évaporation de composants à haut point d'ébullition, et

la colonne de rectification de composants à haut point d'ébullition (12) ou l'évaporateur de composants à haut point d'ébullition (112) est utilisé(e) sous une pression absolue réduite dans la plage de 0,002 à 0,05 bar et à une température de pied dans la plage de 80 à 150 °C, et le courant massique fourni par la sortie de pied de la colonne de rectification de composants purs (11) ou de l'évaporateur de composants purs (111) est introduit dans la zone s'étendant depuis le pied jusqu'au milieu de la colonne de rectification de composants à haut point d'ébullition (12) ou à une entrée d'évaporateur de composants à haut point d'ébullition de l'évaporateur de composants à haut point d'ébullition (112), et

le courant de décharge prélevé par la tête de la colonne de rectification de composants à haut point d'ébullition (12) ou de l'évaporateur de composants à haut point d'ébullition (112) est condensé et envoyé dans la zone s'étendant depuis le pied jusqu'au milieu de la colonne de rectification de composants purs (11) ou à l'entrée d'évaporateur de composants purs de l'évaporateur de composants purs (111).

12. Procédé selon l'une quelconque des revendications précédentes, dans lequel la température de fond dans le réacteur (1) de l'unité de réacteur (24) se situe dans la plage de 100 à 125°C, de préférence dans la plage de 110 à 115°C.

**13.** Procédé selon l'une quelconque des revendications précédentes, dans lequel du cyclohexane est envoyé au réacteur (1) de l'unité de réacteur (24) en une quantité dans la plage de 100 à 600 % en poids, de préférence dans la plage de 200 à 500 % en poids, de façon particulièrement préférée dans la plage de 350 à 450 % en poids, par rapport aux quantités massiques de 2-octanol (13) et d'acide (méth)acrylique (14) envoyées au réacteur (1) de l'unité de réacteur (24).

**14.** Procédé selon l'une quelconque des revendications précédentes, dans lequel la proportion de catalyseur dans le réacteur (1) de l'unité de réacteur (24) est au maximum de 10 % en poids, par rapport à la somme des composants 2-octanol et acide (méth)acrylique présents dans le réacteur (1) de l'unité de réacteur (24).

**15.** Procédé selon l'une quelconque des revendications précédentes, dans lequel les composants arrivant dans le réacteur (1) de l'unité de réacteur (24) sans l'agent d'entraînement cyclohexane présentent les proportions en poids suivantes :

- 2-octanol : 40,00 à 84,39 % en poids
- acide (méth)acrylique : 15,00 à 59,39 % en poids
- catalyseur d'estérification acide : 0,50 à 10,00 % en poids
- inhibiteur de polymérisation : 0,01 à 1,00 % en poids
- composants restants : 0,10 à 5,00 % en poids,

et l'agent d'entraînement cyclohexane est introduit par addition dosée en une quantité telle qu'il en résulte dans le réacteur (1) de l'unité de réacteur (24) une concentration de cyclohexane dans la plage de 10 à 90 % en poids, cette indication de la proportion en % en poids se rapportant aux composants présents dans le réacteur (1) de l'unité de réacteur (24), y compris le cyclohexane,

et/ou les composants s'écoulant au total hors du réacteur (1) de l'unité de réacteur (24) sans l'agent d'entraînement cyclohexane et sans l'eau d'estérification, à savoir le courant liquide résultant issu de la réaction plus la fraction évaporée du mélange réactionnel et évacuée de l'unité de réacteur (24), présentent les proportions en poids suivantes :

- (méth)acrylate de 2-octyle : 50,00 à 95,00 % en poids
- 2-octanol : 1,00 à 30,00 % en poids
- acide (méth)acrylique : 1,00 à 15,00 % en poids
- catalyseur d'estérification acide : 0,50 à 10,00 % en poids
- inhibiteur de polymérisation : 0,01 à 1,00 % en poids
- composants restants : 2,49 à 10,00 % en poids,

et l'azéotrope hétérogène formé par le cyclohexane ainsi que l'eau d'estérification s'écoule hors du réacteur (1) de l'unité de réacteur (24) à une concentration dans la plage de 10 à 50 % en poids, cette indication de la proportion en % en poids se rapportant aux composants s'écoulant au total, y compris le cyclohexane et l'eau d'estérification.

Fig. 1 / 3.

Fig. 2 / 3.

Fig. 3 / 3.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013110877 A1
- WO 2013064775 A1
- WO 2008046000 A1

**In der Beschreibung aufgeführte Nicht-Patentliteratur**

- Ullmann's Encyclopedia of Industrial Chemistry. Electronic Release, 1999